# FASCICULE DE BREVET EUROPEEN

(11) **EP 4 157 311 B1**
(45) Date de publication et mention de la délivrance du brevet: **25.06.2025**
(21) Numéro de dépôt: 21727496.8
(22) Date de dépôt: 28.05.2021
(51) Int. Cl.: A61K 38/16, A61K 38/08, A61K 38/45, A61P 37/04, C07K 16/28, A61K 39/395, A61K 39/00

(54) **COMPLEXE IMMUNOMODULATEUR ET SES APPLICATIONS POUR LA THÉRAPIE**
IMMUNMODULATORISCHER KOMPLEX UND VERWENDUNGEN DAVON ZUR THERAPIE
IMMUNOMODULATORY COMPLEX AND USES THEREOF FOR THERAPY

(30) Priorité: 28.05.2020 FR 2005663
(43) Date de publication de la demande: 05.04.2023
(73) Titulaire: Commissariat à l'Energie Atomique et aux Energies Alternatives, 75015 Paris (FR)
(72) Inventeur: LEONETTI, Michel, 91191 GIF SUR YVETTE CEDEX (FR); SAVATIER, Alexandra, 91191 GIF SUR YVETTE CEDEX (FR); COUSIN, Céline, 91220 BRETIGNY SUR ORGE (FR); SLOBODAN, Culina, 91191 GIF SUR YVETTE CEDEX (FR)
(74) Mandataire: Casalonga
(86) Numéro de dépôt international: PCT/EP2021/064449
(87) Numéro de publication internationale: WO 2021/239996

(56) Documents cités:
- WO-A2-2011/092675
- KNITTEL DELPHINE ET AL: "Heparan sulfates targeting increases MHC class I- and MHC class II-restricted antigen presentation and CD8+T-cell response", VACCINE, ELSEVIER, AMSTERDAM, NL, vol. 34, no. 27, 4 May 2016 (2016-05-04), pages 3093 - 3101, XP029569626, ISSN: 0264-410X, DOI: 10.1016/J.VACCINE.2016.04.073
- WIJKHUISEN A. ET AL: "Production of antigen-specific human IgGs by in vitro immunization", vol. 16, no. 1, 24 February 2016 (2016-02-24), XP055789954, Retrieved from the Internet <URL:https://link.springer.com/content/pdf/10.1186/s12896-016-0253-1.pdf> [retrieved on 20210325], DOI: 10.1186/s12896-016-0253-1
- AIT MEBAREK M ET AL: "Production of human antibodies byin vitroimmunization using a fusion protein containing the transcriptional transactivator of HIV-1", JOURNAL OF IMMUNOLOGICAL METHODS, ELSEVIER SCIENCE PUBLISHERS B.V.,AMSTERDAM, NL, vol. 396, no. 1, 14 August 2013 (2013-08-14), pages 96 - 106, XP028723613, ISSN: 0022-1759, DOI: 10.1016/J.JIM.2013.07.015

## Description

### Domaine technique

L'invention se situe dans le domaine des immunothérapies modulatrices. L'invention concerne un complexe moléculaire consistant en au moins un ligand d'un sucre sulfaté de la famille des glycosaminoglycanes et au moins un ligand d'une autre molécule de surface de cellules présentatrices d'antigène ou de cellules NK ou NKT liés entre eux, pour utilisation en tant que médicament immunostimulant, en particulier en immunothérapie du cancer et des maladies infectieuses.

### Technique antérieure

La majorité des approches thérapeutiques sont fondées sur des médicaments capables d'agir directement sur la cible responsable de la pathologie ou sur les cellules présentant un état non-physiologique. Cependant, des processus pathologiques peuvent être favorisés par des dysfonctionnements du système immunitaire. Des mécanismes d'immunosuppression sont notamment observés lors de la progression tumorale (Galon J. and D. Bruni, Nat. Rev. Drug Discov., 2019 18(3):197-218) ou au cours d'infections par un pathogène infectieux (Wherry E.J. and M. Kurachi, Nature Rev. Immunol., 2015, 15(8) :486-499). Ces observations ont conduit à envisager le développement de médicaments immunothérapeutiques capables de rétablir le fonctionnement adéquat des défenses immunitaires, notamment par modulation de l'activité des lymphocytes T ou de cellules NK ou NKT, afin de permettre au système immunitaire de contrôler plus efficacement les processus pathologiques. Ces immunothérapies modulatrices ont vocation à rétablir ou inhiber la fonctionnalité d'une large partie d'un répertoire lymphocytaire, notamment le répertoire des lymphocytes T-CD4+, T-CD8+ ou T-régulateurs. Elles diffèrent en cela des immunothérapies vaccinales qui ont vocation à induire un nombre restreint de lymphocytes correspondant aux cellules spécifiques de(s) l'Ag(s) inclus dans le vaccin.

Pour développer des médicaments immunothérapeutiques, il faut préalablement identifier des cibles jouant un rôle crucial dans le contrôle de la réponse immunitaire. Dans le courant des années 90, il a été montré que des molécules exprimées à la surface des lymphocytes T, respectivement appelées PD-1 et CTLA-4, peuvent induire des signaux inhibiteurs régulant négativement l'activité de ces cellules et peuvent ainsi réguler certains mécanismes de défense immunitaire (Ishida, Y., Agata, Y., Shibahara, K., & Honjo, T. (1992). EMBO J., 11(11), 3887-3895.; Freeman, et al. (2000). J Exp Med, 192(7), 1027-1034.; Krummel MF, Allison JP, J. Exp. Med., 1995 Aug 1;182(2):459-65). Ces deux molécules ont été appelées points de contrôle immunitaire (« immune-check point »), molécules de points de contrôle immunitaire inhibitrices, ou ICP inhibiteurs.

Les mécanismes moléculaires responsables de l'activité de ces ICP inhibiteurs ont été étudiés. Il a notamment été observé que PD-1 et CTLA-4 interagissent avec des ligands exprimés à la surface de cellules présentatrices de l'antigène (CPAg) ou de cellules tumorales. Ces ligands sont respectivement appelés PD-L1 et B7. L'interaction PD-1/PD-L1 ou CTLA-4/B7 médie alors des signaux d'inhibition des lymphocytes T et provoque de ce fait différents mécanismes d'immunosuppression. Il a de plus été montré que des anticorps (Ac) spécifiques de PD-1, PD-L1 ou CTLA-4 peuvent bloquer l'interaction PD-1/PD-L1 ou CTLA-4/B7 permettant ainsi la levée de l'inhibition et la réactivation des lymphocytes T (Hodi, F.S. et al., PNAS. (2003), 100(8), 4712-4717; Iwai, Y. et al., Int. Immunol., (2005). 17(2), 133-144). Certains de ces Ac immunomodulateurs se sont avérés capables de limiter la croissance de différents cancers (mélanome, poumon, etc) et d'accroître significativement l'espérance de vie des patients. Ils sont maintenant couramment utilisés en tant que médicament immunothérapeutique anti-tumoral chez l'homme (Adachi K. and K. Tamada, Cancer Sci., 2015;106(8):945-50; Riley RS et al., Nat. Rev. Drug Discov. 2019 18(3):175-196). Ils sont aussi envisagés pour d'autres domaines thérapeutiques, notamment pour le traitement de maladies infectieuses (Rao M. et al., Int. J. Infect. Dis., 2017;56:221-228). Ces premiers anticorps anti-ICP inhibiteurs présentent cependant l'inconvénient de ne fonctionner que chez 10 à 30% des patients atteints d'un cancer (Pitt JM et al., Immunity. 2016 Jun 21;44(6):1255-69). De nombreux groupes de recherche ont donc pour objectif de découvrir de nouveaux composés immunomodulateurs plus efficaces et/ou utilisables en combinaison avec les immunothérapies décrites précédemment. Comme plusieurs nouveaux ICP inhibiteurs ont été identifiés, des travaux sont focalisés sur la sélection de composés capables de lier ces ICP ou leurs ICP-ligand et de neutraliser ainsi la liaison ICP/ICP-ligand afin de réactiver les lymphocytes T. La découverte d'ICP activateurs exprimés à la surface de la cellule T a quant à elle conduit à focaliser d'autres travaux sur la sélection de ligands agonistes de ces ICP (Mahoney KM. Et al., Nat. Rev. Drug Discov., 2019, 14 :561-584 ; De Sousa Linhares A., Front. Immunol., 2018, 31;9:1909. ; Granier C. et al, ESMO Open, 2017 Jul 3;2(2):e000213).

Pour immunomoduler le plus efficacement possible la réponse immunitaire on cherche à sélectionner des molécules thérapeutiques capables de lier des ICP ou IC P-ligand exprimés sélectivement à la surface de cellules immunitaires effectrices, comme les lymphocytes T et les cellules NK ou NKT, ou de cellules présentatrices de l'Ag (CPAg). En effet, cette sélectivité d'expression permet de limiter la dissémination de la molécule vers des cellules non-immunes. Il en résulte ainsi une augmentation de l'efficacité thérapeutique et une diminution du risque d'effets secondaires.

Le glycocalyx est notamment constitué par les protéoglycanes qui sont des glycoprotéines comprenant une ou plusieurs chaînes de glycosaminoglycanes (GAG) non-ramifiées. Parmi ceux-ci, la famille des héparanes sulfates protéoglycanes (HSPG) correspond à des protéines associées à des GAG sulfatés: les héparanes sulfates (HS). Les HSPG, qui jouent un rôle central dans de nombreux processus biologiques (prolifération cellulaire, adhésion cellulaire, inflammation, coagulation, pénétration cellulaire de microorganismes pathogènes, en particulier des virus et des parasites), sont retrouvées à la surface de la plupart des cellules de mammifères et dans les matrices extracellulaires (Dreyfuss et al., Annuals of the Brazilian Academy of Sciences, 2009, 81, 409-429). Cette expression ubiquitaire conduit donc à considérer que les HSPG, et leurs domaines HS, ne représentent pas des cibles immunomodulatrices pertinentes. De plus, la capacité de ligands d'HS à induire l'activation de cellules immunitaire n'a pas été identifiée à ce jour.

### Résumé de l'invention

Lors de leurs travaux, les inventeurs ont tout d'abord découvert qu'un ligand d'un sucre sulfaté de la famille des GAG, les héparanes sulfates (HS), est incapable d'activer des cellules dendritiques *in vitro.* Ils ont ensuite observé qu'un complexe moléculaire contenant ce ligand et un ligand d'une molécule de surface de cellules présentatrices de l'antigène (CPAg) potentialise l'effet immunomodulateur du ligand des héparanes sulfates (HS). En effet, ce complexe moléculaire induit encore plus fortement la CPAg comme le montre l'augmentation de la sécrétion des cytokines IL-6 et IL-12, et permet l'activation subséquente de lymphocytes T. Les inventeurs ont montré que l'association entre les deux ligands peut être réalisée sous la forme d'un complexe covalent ou non covalent, notamment sous la forme d'une protéine de fusion. Les inventeurs ont de plus observé que les propriétés immunomodulatrices de ce complexe permettent de mieux contrôler des processus pathologiques et, notamment, de ralentir la progression d'une tumeur.

Ces résultats sont surprenants car, bien que décrit comme pouvant jouer un rôle de récepteur ou de corécepteur, il n'avait jamais été montré que les HSPG peuvent potentialiser l'activation médiée par un ligand de récepteur exprimé spécifiquement à la surface des CPAgs. Enfin, le complexe moléculaire, qui cible les HS d'expression ubiquitaire, pouvait disséminer vers la matrice extracellulaire ou vers des cellules dépourvues d'intérêt pour la pathologie. Il ne devait donc théoriquement pas être capable de moduler l'activité des CPAg et d'un large répertoire de lymphocytes T avec suffisamment d'efficacité pour qu'un effet immunothérapeutique se produise.

Les inventeurs ont aussi découvert que les résultats obtenus avec les CPAg étaient transposables à d'autres cellules de la réponse immunitaire innée, les cellules NK et NKT. Ainsi, les inventeurs ont montré qu'un ligand d'une molécule de surface de cellules NK ou NKT incapable d'induire à lui seul ces cellules devient capable de d'augmenter le nombre de cellules activées lorsqu'il est associé, dans un complexe moléculaire, à un ligand d'un sucre sulfaté de la famille des GAG.

En conséquence, la présente invention a pour objet un complexe moléculaire pour utilisation en tant que médicament immunomodulateur, de préférence immunostimulant, ledit complexe consistant en au moins un ligand d'un sucre sulfaté de la famille des glycosaminoglycanes (premier ligand ou L1) et au moins un ligand d'une autre molécule de surface de CPAg ou de cellule NK ou NKT (deuxième ligand ou L2) liés entre eux, et ledit complexe étant dépourvu d'un antigène spécifique de la maladie à traiter.

Selon des modes de réalisations préférés de l'invention, le premier ligand est un peptide de liaison des héparanes sulfates sélectionné dans le groupe constitué par : un peptide issu de la protéine Tat du VIH comprenant au moins la région basique Tat 49-57 (SEQ ID NO : 3) tel que les peptides Tat 49-57 (SEQ ID NO: 3), Tat37-57 (SEQ ID NO : 8) et Tat22-57C(22-37)S (SEQ ID NO 9) ; un peptide polyarginine R7 à R11 ; et un peptide issu du domaine R de la toxine diphtérique comprenant le domaine R de la toxine diphtérique (SEQ ID NO : 5) ou au moins le fragment DT453-467 (SEQ ID NO 7) dudit domaine qui comprend la région de liaison des héparanes sulfates.

Selon des modes de réalisations préférés de l'invention, le deuxième ligand cible une molécule de surface des CPAgs sélectionnée dans le groupe constitué par : les récepteurs lectines de type C, les immunoglobulines membranaires, les récepteurs pour la région constante des immunoglobulines, et les molécules de points de contrôle immunitaire (ICP).

Selon des modes de réalisations préférés de l'invention, le deuxième ligand cible une molécule de surface de cellules NK ou NKT sélectionnée dans le groupe constitué par : les récepteurs NKG2D, NKp30, NKp44, NKp46, NKp80, Ly49H, les récepteurs KIR, NKG2A, et les ICP PD-1, CTLA-4, TIM-3, TIGIT, LAG-3.

De préférence, le deuxième ligand est sélectionné dans le groupe constitué par : (i) les anticorps dirigés contre lesdites molécules de surface des CPAgs ou des cellules NK ou NKT et leurs fragments contenant au moins le paratope ; (ii) les immunoglobulines de préférence les IgG, et leurs fragments comprenant au moins la région Fc ; et (iii) les protéines et les fragments de protéines qui lient la région Fc et/ou Fab des anticorps, notamment la protéine A de *S. aureus,* son fragment BB (SEQ ID NO : 1) et son dérivé ZZ (SEQ ID NO : 2).

Selon des modes de réalisations préférés de l'invention, ledit complexe est sous forme d'oligomères ou d'un mélange de monomères et d'oligomères.

Selon des modes de réalisations préférés de l'invention, ledit complexe consiste en une protéine de fusion entre le premier et le deuxième ligand. Un type de complexe plus particulièrement préféré selon l'invention consiste en une protéine de fusion comprenant un premier ligand choisi parmi : Tat49-57 (SEQ ID NO : 3), Tat37-57 (SEQ ID NO : 8), Tat22-57C(22-37)S (SEQ ID NO 9), le domaine R de la toxine diphtérique (SEQ ID NO : 5) ou le fragment DT453-467 (SEQ ID NO 7), et un deuxième ligand choisi parmi : le fragment BB de la protéine A (SEQ ID NO : 1) ou son dérivé ZZ (SEQ ID NO : 2).

Selon d'autres modes de réalisations préférés de l'invention, le deuxième ligand est un anticorps ou un fragment d'anticorps et le premier ligand forme une protéine de fusion avec un élément de liaison aux immunoglobulines, de préférence la protéine A de *S. aureus,* son fragment BB (SEQ ID NO : 1) ou son dérivé ZZ (SEQ ID NO : 2). De préférence, ladite protéine de fusion comprend un premier ligand choisi parmi Tat49-57 (SEQ ID NO : 3), Tat37-57 (SEQ ID NO : 8), Tat22-57C(22-37)S (SEQ ID NO 9), le domaine R de la toxine diphtérique (SEQ ID NO : 5) ou le fragment DT453-467 (SEQ ID NO 7) et un élément de liaison aux immunoglobulines choisi parmi le fragment BB de la protéine A (SEQ ID NO : 1) ou son dérivé ZZ (SEQ ID NO : 2). Dans un type de complexe plus particulièrement préféré selon l'invention, l'élément de liaison aux immunoglobulines est le fragment BB de la protéine A (SEQ ID NO : 1), et ladite protéine de fusion étant complexée au deuxième ligand qui consiste en une immunoglobuline entière. Dans un autre type de complexe plus particulièrement préféré selon l'invention, ladite protéine de fusion est complexée au deuxième ligand qui est choisi parmi un anticorps anti-RFcgamma I, II et/ou III, anti-DEC-205, anti-DC-SIGN, anti-CD74, anti-CD275, anti-CD335, anti-CD336, anti-CD56, anti-CTLA-4, anti-PD-L1, anti-OX40 ou un fragment des anticorps précédents comprenant au moins le paratope.

Selon des modes de réalisations préférés de l'invention, le complexe est pour une utilisation en tant que médicament immunostimulant, de préférence pour activer des cellules présentatrices de l'antigène, notamment des cellules dendritiques ou des monocytes, pour activer des cellules NK ou NKT, et/ou pour activer la sécrétion des cytokines IL-6 et/ou IL-12.

La présente invention a également pour objet une composition pour utilisation en tant que médicament immunomodulateur, de préférence immunostimulant, comprenant au moins un complexe moléculaire selon l'invention et au moins un véhicule pharmaceutiquement acceptable, une substance porteuse et/ou un adjuvant. De préférence, l'adjuvant est un oligodéoxynucléotide CpG, de l'acide polyinosinique-polycytidylique ou un mélange d'oligodéoxynucléotide(s) CpG, et d'acide polyinosinique-polycytidylique, et/ou la substance porteuse est une nanoparticule.

Selon des modes de réalisations préférés de l'invention, ladite composition comprend au moins un autre agent thérapeutique, de préférence au moins un inhibiteur de point de contrôle immunitaire, de manière préférée un anti-PD-1, un anti-PD-L1 ou un anti-CTLA-4

Selon des modes de réalisations préférés de l'invention, la composition est pour utilisation en immunothérapie du cancer ou des maladies infectieuses.

### Exposé de l'invention

La présente invention a pour objet un complexe moléculaire pour utilisation en tant que médicament immunostimulant en immunothérapie du cancer ou des maladies infectieuses, ledit complexe consistant en au moins un ligand d'un sucre sulfaté de la famille des glycosaminoglycanes exprimé à la surface de cellules présentatrices d'antigène ou de cellules NK ou NKT (premier ligand) et au moins un ligand d'une autre molécule de surface de CPAg ou de cellules NK ou NKT (deuxième ligand) liés entre eux, et ledit complexe étant dépourvu d'un antigène spécifique de la maladie à traiter.

Un tel complexe est capable d'induire des cellules du système immunitaire dans un sens favorable au déclenchement de la réponse immunitaire. Ainsi, lorsque des splénocytes sont incubés avec ces complexes on observe l'activation de cellules dendritiques qui deviennent capables de sécréter des cytokines IL-6 et IL-12. De plus, l'induction des DC peut être obtenue pour des seconds ligands qui sont incapables de déclencher un tel effet lorsqu'ils sont non associés, comme la forme isolée du ligand de CPAg appelé ZZ. L'association du ligand des HS peut aussi permettre de potentialiser l'effet d'un ligand capable, sous forme isolée, d'activer des CPAg tel qu'un Ac polyclonal humain non-spécifique. L'association du ligand des HS à un ligand de cellules NK ou NKT peut aussi permettre d'activer ces types cellulaires alors qu'il est dépourvu de cette capacité sous forme isolée. Enfin, un complexe immunomodulateur, de préférence immunostimulant, selon l'invention peut induire un effet thérapeutique, en particulier antitumoral, supérieur à celui obtenu avec un Ac anti-ICP (anti-PD1), notamment lorsque la composition comprend un adjuvant.

### Définitions

On entend par « cellules immunitaires de la réponse innée », les cellules dendritiques, les cellules NK et NKT, les granulocytes (mastocytes, polynucléaires neutrophiles, éosinophiles et basophiles) et les phagocytes (monocytes, macrophages, polynucléaires neutrophiles etc).

On entend par « cellule présentatrice d'antigène», cellule présentatrice de l'Ag, CPA, CPAg ou APC pour *Antigen Presenting Cell,* une cellule exprimant une ou plusieurs molécules du complexe majeur d'histocompatibilité (CMH) de classe I et de classe II (molécules HLA de classe I et de classe Il chez l'homme) et capable de présenter les Ags aux lymphocytes T CD4+ et T CD8+ spécifiques de cet Ag. Comme cellules présentatrices d'Ag, on peut citer notamment les cellules dendritiques (CD ou DC pour *Dendritic Cell*), les monocytes, les macrophages, les lymphocytes B, les lignées lymphoblastoïdes, et les lignées de cellules humaines ou animales génétiquement modifiées exprimant des molécules du CMH de classe I et de classe II, notamment des molécules HLA I et HLA II.

On entend par cellule NK un lymphocyte granuleux exprimant les molécules CD56 et CD16 et doté d'une activité cytotoxique qui ne nécessite pas l'exposition préalable à l'Ag.

On entend par cellule NKT un lymphocyte granuleux exprimant des marqueurs de cellule NK, notamment les molécules CD56 et CD16 et des marqueurs de lymphocytes T, notamment la molécule CD3. Cette cellule est dotée d'une activité cytotoxique qui ne nécessite pas l'exposition préalable à l'Ag.

On entend par « molécule de surface de CPAg », une molécule exprimée à la surface de cellules présentatrices d'Ag.

On entend par « molécule de surface de cellule NK ou NKT », une molécule exprimée à la surface de cellules NK ou NKT.

On entend par « molécule de surface spécifique des CPAg », une molécule exprimée essentiellement sur les cellules présentatrices d'Ag, c'est-à-dire exprimée sur un nombre très limité de cellules autres que les CPAg. Il s'agit donc d'une molécule possédant une spécificité d'expression élevée pour les CPAg.

On entend par « molécule de surface spécifique de cellule NK ou NKT », une molécule exprimée essentiellement sur les cellules NK ou NKT, c'est-à-dire exprimée sur un nombre très limité de cellules autres que les de cellule NK ou NKT. Il s'agit donc d'une molécule possédant une spécificité d'expression élevée pour les cellules NK ou NKT.

On entend par glycosaminoglycane (GAG) un polysaccharide linéaire composé de la répétition d'un diholoside contenant toujours une hexosamine (glucosamine (GlcN) ou galactosamine (GaIN)) et un autre ose (acide glucuronique (GlcA), acide iduronique (IdoA), galactose (Gal)). La glucosamine est soit N-sulfatée (GlcNS), soit N-acétylée (GlcNac). La galactosamine est toujours N-acétylée (GalNac). Les glycosaminoglycanes sulfatés sont notamment des polymères simples de GIcA tel que le chondroïtine sulfate et des copolymères comprenant à la fois des résidus de GIcA et/ou d'IdoA et/ou de Gal, tels que l'héparine, l'héparane sulfate, le dermatane sulfate, et le kératane sulfate. Les chaînes de GAG peuvent être liées de façon covalente à des protéines (protéoglycanes) qui sont exprimées à la surface des cellules de mammifères et/ou sécrétées dans le milieu extracellulaire. Le premier ligand selon l'invention se lie à un sucre sulfaté de la famille des glycosaminoglycanes qui est exprimé à la surface des cellules de mammifères, incluant entre autre les CPAg et les cellules NK ou NKT.

**On** entend par « Ac », une immunoglobuline (IgG, IgM, IgA, IgD, IgE). Le terme Ac désigne un Ac spécifique, c'est à dire un Ac dirigé contre une molécule x particulière (Ac anti-molécule x) notamment une molécule de surface de CPAg (Ac anti-molécule de surface de CPAg. Le terme immunoglobuline désigne un Ac non-spécifique.

On entend par « individu », un individu humain ou animal, de préférence humain.

On entend par « ligand » d'une molécule, tout agent capable de lier cette molécule avec une affinité suffisamment élevée pour former un complexe stable, *in vitro* et *in vivo.*

On entend par « ligand de CPAg », un ligand d'une molécule de surface de cellules présentatrices d'Ag.

On entend par « ligand de cellules NK ou NKT», un ligand d'une molécule de surface de cellules NK ou NKT.On entend par « ligand d'héparane sulfate » un agent qui lie l'héparine avec un signal de densité optique au moins égal à 50% du signal mesuré quand ZZ-Tat_{22-57C(22-37)S} est incubé à 100nM pH7,2 dans le test ELISA de l'exemple 1.

On entend par « antigène » toute substance pouvant être reconnue spécifiquement par le système immunitaire et en particulier par les anticorps et les cellules du système immunitaire (lymphocytes B, lymphocytes T CD4+, lymphocytes T CD8+) et capable d'induire une réponse immunitaire spécifique
On entend par « antigène spécifique de la maladie à traiter » un antigène qui induit une réponse immunitaire dirigée spécifiquement contre la maladie à traiter. Ladite réponse immunitaire spécifique de la maladie à traiter inclut la production d'anticorps, et/ou l'induction d'une réponse T cytotoxique (activation de lymphocytes T CD8+) ou T auxiliaire (activation de lymphocytes T CD4+) dirigés contre un antigène d'un pathogène ou d'une cellule tumorale responsables de ladite maladie à traiter.

On entend par « immunomodulateur » un agent capable de contrôler la réponse immunitaire et notamment de réguler, de façon positive ou négative, la réponse relative de différentes populations ou sous-populations de cellules immunitaires telles que les lymphocytes T et B, les CPAg, les cellules NK ou NKT. Le terme immunomodulateur englobe les termes immunosuppresseur et immunostimulant. On entend par « immunostimulant » un agent capable de réguler de façon positive, c'est-à-dire d'activer, la réponse relative de différentes populations ou sous-populations de cellules immunitaires telles que les lymphocytes T et B, les CPAg, les cellules NK ou NKT. L'effet de l'immunomodulateur, de préférence immunostimulant, s'exerce sur un large répertoire cellulaire. Il est indépendant de la présence d'un antigène spécifique de la pathologie à traiter, et ce contrairement à un vaccin qui nécessite la présence d'un antigène spécifique de la maladie à traiter et induit une réponse immunitaire spécifique dudit antigène. Alors que l'utilisation d'un immunogène ou d'un vaccin est limitée à la maladie comprenant l'antigène spécifique de cette maladie, le complexe immunomodulateur, de préférence immunostimulant selon l'invention qui est indépendant de la présence d'un antigène spécifique de la pathologie à traiter est utilisable en immunothérapie de nombreuses maladies telles que le cancer et les maladies infectieuses.

On entend par « peptide », une séquence d'acides aminés naturels ou synthétiques, éventuellement modifiée. Le terme peptide est utilisé indépendamment de la taille de la séquence d'acides aminés.

Le complexe moléculaire selon l'invention consiste en au moins deux ligands de molécules de surface de CPAg ou de cellule NK ou NKT liés entre eux : le premier ligand, dénommé L1, qui cible un GAG sulfaté et le deuxième ligand, dénommé L2, qui cible une autre molécule de surface de CPAg. Le complexe moléculaire selon l'invention peut comprendre un ou plusieurs ligands L1 et un ou plusieurs ligands L2 liés entre eux. Selon certains modes de réalisation préférés de l'invention, le complexe moléculaire consiste en un ligand L1 lié à un ligand L2.

Le complexe moléculaire selon l'invention, de préférence un complexe immunostimulant, est dépourvu d'antigène spécifique de la maladie à traiter, tel qu'un antigène vaccinal spécifique de la pathologie à traiter. Lorsque l'un des ligands du complexe selon l'invention comprend un antigène spécifique d'une maladie à traiter, alors le traitement de ladite maladie avec ledit complexe est exclue de l'invention.

Les ligands de CPAg, de cellule NK ou NKT sont des molécules ou des complexes de molécules, naturels, recombinants ou synthétiques, de nature protéique (protéine, peptide, polypeptide), lipidique, glucidique, acide nucléique ou mixte (glycolipide, glycoprotéine, lipoprotéine). Selon certains modes de réalisation préférés de l'invention, le premier et le deuxième ligand sont des protéines, polypeptides ou peptides, désignés ci-après « peptides », de préférence recombinants ou synthétiques, Les protéines, polypeptides ou peptides recombinants sont avantageusement produits en cellules procaryotes ou eucaryotes, dans un système d'expression approprié, notamment à la production de protéines thérapeutiques. Par exemple, les protéines, polypeptides ou peptides recombinants peuvent être produits chez *E. coli* ou en cellules HEK ou CHO.

Le premier et le deuxième ligand sont associés ou liés l'un à l'autre par tout moyen adapté. Ils peuvent être liés de façon covalente ou non-covalente, soit directement, soit par l'intermédiaire d'un élément de liaison et forment ainsi un complexe moléculaire. Le complexe moléculaire ou complexe est constitué de deux ligands ou plus, et éventuellement d'élément(s) de liaison approprié(s).

La liaison ou association covalente des ligands est notamment générée par un couplage chimique covalent (formation d'un conjugué covalent) ou par la construction d'une protéine de fusion (fusion génétique).

Le complexe immunomodulateur, de préférence un complexe immunostimulant, est sous forme monomérique, oligomérique ou mixte (mélange de monomères et d'oligomères). Selon certains modes de réalisation préférés de l'invention, ledit complexe est sous forme oligomérique ou mixte.

Selon certains modes de réalisation préférés de l'invention, le complexe moléculaire immunomodulateur, de préférence un complexe immunostimulant, consiste en une protéine de fusion entre le ou les premier(s) (L1) et le ou les deuxième(s) ligand (L2). Les séquences en acides aminés de L1 et L2 sont fusionnées dans l'ordre approprié, soit directement, soit par l'intermédiaire d'un peptide espaceur approprié. En fonction des tailles respectives des séquences en acides aminés de L1 et L2, elles sont soit fusionnées au niveau de leurs extrémités (extrémité N-terminale de l'une des séquences fusionnée à l'extrémité C-terminale de l'autre séquence) ou l'une des séquences est insérée dans l'autre séquence à un site approprié qui n'a pas d'effet délétère sur la liaison du ligand à son récepteur exprimé à la surface des CPAgs, des I cellules NK ou NKT.

La liaison non-covalente est notamment générée par adsorption sur une nanoparticule. Elle peut aussi être obtenue en utilisant une molécule (élément de liaison), possédant une affinité élevée et spécifique pour L1 ou L2. Cet élément de liaison est lié de façon covalente à l'un des ligands pour associer de façon non-covalente l'autre ligand. Lorsque l'un des ligands est un anticorps (Ac), l'élément de liaison est notamment une protéine ou un fragment de protéine qui lie la région Fc et/ou Fab des immunoglobulines, tels que décrits dans la Demande FR 2759296. De tels éléments de liaison aux immunoglobulines incluent notamment la protéine A de *S. aureus,* son fragment BB (SEQ ID NO : 1) et son dérivé ZZ (SEQ ID NO : 2), les deux premières protéines liant les régions Fc et le Fab des immunoglobulines alors que ZZ ne lie que la région Fc. Par exemple, lorsque l'un des ligands est un Ac ou un fragment d'anticorps, l'élément de liaison aux immunoglobulines est lié de façon covalente (couplage chimique covalent ou protéine de fusion) à l'autre ligand. L'élément de liaison peut aussi se lier à des partenaires respectivement couplés à L1 et L2, par exemple, l'élément de liaison est la Streptavidine qui se lie à des ligands L1 et L2 biotinylés (L1-biot/Streptavidine/biot-L2). L'affinité de l'élément de liaison pour son partenaire, dans le complexe L1-L2 est suffisante pour qu'il ne se dissocie pas immédiatement de ce complexe *in vivo.*

Selon certains modes de réalisation préférés du complexe immunomodulateur de l'invention, de préférence un complexe immunostimulant, l'un des ligands est un anticorps ou un fragment d'anticorps et l'autre ligand forme une protéine de fusion avec un élément de liaison aux immunoglobulines, de préférence la protéine A de *S. aureus,* son fragment BB (SEQ ID NO : 1) ou son dérivé ZZ (SEQ ID NO : 2). De préférence, le deuxième ligand est un anticorps ou un fragment d'anticorps et le premier ligand forme une protéine de fusion avec un élément de liaison aux immunoglobulines, de préférence la protéine A de *S. aureus,* son fragment BB (SEQ ID NO : 1) ou son dérivé ZZ (SEQ ID NO : 2).

Les propriétés immunomodulatrices, de préférence immunostimulantes du complexe selon l'invention sont évaluées par des tests immunologiques classiques connus de l'homme du métier tels que ceux décrits dans les exemples. Les propriétés immunostimulantes sont notamment évaluées par l'analyse de l'expansion et/ou de l'activation de différentes populations ou sous-populations de cellules immunitaires telles que les lymphocytes T CD4+, T-CD8+ et B ; les monocytes, les cellules dendritiques incluant les cellules dendritiques conventionnelles (cDC) et plasmacytoïdes (pDC), les cellules NK, les cellules NKT. L'analyse de l'expansion est notamment réalisée en cytométrie de flux à l'aide de marqueurs appropriés pour les différentes populations de cellules à analyser. L'analyse de l'activation des cellules immunitaires peut être réalisée par la détection de marqueurs d'activation (CD69), et/ou de maturation (CD86) en cytométrie de flux ou par dosage de cytokines sécrétées dans le milieu extracellulaire, en particulier l'IL-6 et/ou IL-12, par des tests classiques tels que l'ELISA. Comme mentionné ci-dessus, l'effet l'immunomodulateur, de préférence immunostimulant, du complexe moléculaire selon l'invention est indépendant de la présence d'un antigène spécifique de la pathologie à traiter.

Le complexe moléculaire selon l'invention, de préférence un complexe immunostimulant, se lie à au moins un sucre sulfaté de la famille des glycosaminoglycanes exprimé à la surface des CPAgs, NK ou NKT(GAG sulfaté) et une autre molécule de surface de CPAg, NK ou NKT, en particulier une molécule de surface spécifique des CPAgs, NK ou NKT. Le GAG sulfaté qui est ciblé par le premier ligand est de préférence un héparane sulfate, un chondroïtine sulfate, un dermatane sulfate, ou un kératane sulfate, de manière préférée un héparane sulfate. Le ligand de GAG sulfaté (premier ligand) peut être issu d'une cellule de mammifère ou d'un microorganisme pathogène, notamment un virus (Adénovirus, Cytomégalovirus, VIH, Virus Sindbis), une bactérie (*Mycobacterium bovis, Bordetella pertussis*), un parasite (*Leishmania sp.*) ou une toxine, il s'agit en particulier d'un complexe moléculaire, d'une molécule ou un de ses fragments qui lie l'héparine, et/ou les héparanes sulfates. De tels ligands sont notamment décrits dans « Heparan Binding Proteins", H. Edward Conrad, Academic Press, San Diego *and London* et Dreyfuss et al., Annuals of the Brazilian Academy of Sciences, 2009, 81, 409-429 (voir notamment le Tableau II). Des exemples de ces ligands incluent de façon non-limitative : des ligands endogènes de GAG (Thrombine, Urokinase, Vitronectine, facteur de croissance des fibroblastes et autres), la protéine Tat du VIH (SEQ ID NO : 10) et ses fragments, en particulier les fragments comprenant uniquement la région basique de Tat (Tat49-57 (SEQ ID NO : 3)) ou la région basique et la région centrale de Tat (core ; Tat38-48 (SEQ ID NO : 4)) ; les dodécaèdres dérivés du penton de l'Adénovirus (Vivès et al., Virology, 2004, 321 : 332-340) ; la protéine d'enveloppe du VIH ou la région V3 de cette protéine (Roderiquez et al., J. Virol., 1995, 69, 2233-), la glycoprotéine d'enveloppe du virus Sindbis (Byrnes, A.P. et Griffin, D.E., J. Virol., 1998, 2, 7349-7356) et le domaine R de la toxine diphtérique (DTR ou DTRBD ; fragment 382 à 535 de DT (SEQ ID NO : 5)) ; Lobeck et al., Infection and Immunity, 1998, 66, 418-423). On peut citer également les peptides de pénétration cellulaire (*CPP pour Cell Penetrating Peptide*) qui lient l'héparine, les héparanes sulfates et/ou les chondroïtines sulfates tels que les peptides très riches en résidus basiques, en particulier en arginines qui incluent les peptides dérivés de la région basique de la protéine Tat du VIH (Tat49-57), précités, et les peptides polyarginines (R7 à R11), ainsi que les peptides basiques/amphiphiles tels que les peptides dérivés de l'homéodomaine de la protéine Antennapedia (pénétratine ; fragment 43-58 (SEQ ID NO : 6)).

Alternativement, le premier ligand est un Ac naturel ou recombinant dirigé contre un GAG sulfaté, de préférence l'héparine, les héparanes sulfates ou les chondroïtines sulfates ou un fragment de cet Ac contenant au moins le paratope (domaine de liaison à l'Ag) tel qu'un fragment Fab, Fab', F(ab')₂, Fv ou Fv simple chaîne (scFv pour *single-chain Fv*), Fabc, et fragment Fab comprenant une portion du domaine Fc. De tel Ac sont notamment décrits dans Thompson et al., J. Biol. Chem., 2009, 284, 35621-35631 et van Kuppevelt et al., J. Biol. Chem., 1998, 273, 12960-12966. De préférence, ledit Ac ou fragment d'Ac est humain ou humanisé.

Selon des modes de réalisation avantageux du complexe moléculaire, de préférence immunostimulant, de l'invention, le premier ligand est un peptide de liaison des héparanes sulfates sélectionné dans le groupe constitué par : un peptide issu de la protéine Tat du VIH, comprenant au moins la région basique Tat49-57 (SEQ ID NO : 3) tel que les peptides Tat 49-57 (SEQ ID NO: 3), Tat37-57 (SEQ ID NO : 8) et Tat22-57C(22-37)S (SEQ ID NO 9) ; un peptide polyarginine R7 à R11 ; et un peptide isssu du domaine R de la toxine diphtérique comprenant le domaine R de la toxine diphtérique (SEQ ID NO : 5) ou au moins le fragment DT453-467 (SEQ ID NO 7) dudit domaine qui comprend la région de liaison des héparanes sulfates. De manière préférée, le premier ligand est un peptide de liaison des héparanes sulfates sélectionné dans le groupe constitué par : un peptide issu de la protéine Tat du VIH comprenant au moins la région basique Tat 49-57 (SEQ ID NO : 3), en particulier le peptide Tat22-57C(22-37)S (SEQ ID NO 9) et un peptide comprenant le domaine R de la toxine diphtérique (SEQ ID NO : 5).

Dans des modes de réalisation avantageux du complexe moléculaire, de préférence immunostimulant, de l'invention, lorsque le complexe comprend le domaine R de la toxine diphtérique (DTR : SEQ ID NO : 5), alors le domaine est produit en cellules de mammifères, en particulier sous forme d'une protéine de fusion avec le deuxième ligand ou un élément de liaisons aux immunoglobulines, de façon à former des oligomères.

L'autre molécule exprimée à la surface des CPAg qui est ciblée par le deuxième ligand est aussi bien une molécule de surface ubiquitaire autre qu'un GAG sulfaté ; une molécule de surface exprimée essentiellement sur les cellules immunitaires de la réponse innée ou adaptative incluant les CPAg, c'est-à-dire une molécule de surface spécifique des cellules immunitaires de la réponse innée ou adaptative incluant les CPAg ; ou une molécule de surface exprimée essentiellement sur les CPAg, et en particulier sur les cellules dendritiques, c'est-à-dire une molécule de surface spécifique des CPAg et en particulier des cellules dendritiques. Parmi ces molécules de surface exprimées essentiellement sur les CPAgs et en particulier sur les cellules dendritiques, on peut citer notamment : les molécules du CMH de classe II, en particulier les chaînes α, β et la chaîne γ ou chaîne invariante (li, fragment li ou CD74) des molécules CMH Il ; les immunoglobulines de surface ou immunoglobulines membranaires ; des intégrines telles que notamment CD11c et MAC1 ; les récepteurs de la transferrine, les récepteurs lectines de type C tels que notamment le (récepteur du mannose (CD206), DEC-205 (CD205), CD206, DC-SIGN (CD209), LOX1, Dectin-1 (récepteur du béta-glucane), Dectin-2, Clec9A, Clec12A, DCIR2, FIRE et CIRE ; les récepteurs pour la région constante des immunoglobulines (FcR ou RFc), notamment FcγR tel que FcyRI (CD64), FcγRII (CD32) et FcγRIII (CD16) ; la superfamille des récepteurs du TNF tel que CD40 ; et les récepteurs du complément. Parmi les molécules de surface exprimées essentiellement sur les CPAgs, ont peut citer les molécules de points de contrôle immunitaire (ICP) et leurs ligands (ICP-ligands) exprimés sur les CPAg, tels que de façon non-limitative PDL1, PDL2, CD155, CD80, CD86, CD40, OX40L, ICOSL (CD275), CD70 (Wykes MN, Nat. Rev. Immunol., 2018, 18:91-104).

L'autre molécule exprimée à la surface des cellules NK ou NKT qui est ciblée par le deuxième ligand est aussi bien une molécule de surface ubiquitaire autre qu'un GAG sulfaté ; une molécule de surface exprimée essentiellement sur les cellules immunitaires de la réponse innée ou adaptative incluant les cellules NK ou NKT, c'est-à-dire une molécule de surface spécifique des cellules immunitaires de la réponse innée ou adaptative incluant les cellules NK ou NKT; ou une molécule de surface exprimée essentiellement sur les cellules NK ou NKT c'est-à-dire une molécule de surface spécifique des cellules NK ou NKT. Parmi ces molécules de surface exprimées essentiellement sur les cellules NK ou NKT, on peut citer notamment : NKG2D, NKp30, NKp44, NKp46, NKp80, CD56, CD16, les récepteurs KIR, NKG2A, et les ICP PD-1, CTLA-4, TIM-3, TIGIT, LAG-3 et OX40.

Selon des modes de réalisation avantageux du complexe moléculaire, de préférencer immunostimulant, de l'invention, le deuxième ligand cible une molécule de surface exprimée essentiellement sur les CPAg, et en particulier sur les cellules dendritiques, c'est-à-dire une molécule de surface spécifique des CPAg et en particulier des cellules dendritiques, de préférence sélectionnée dans le groupe constitué par : les récepteurs lectines de type C, les immunoglobulines membranaires, les récepteurs pour la région constante des immunoglobulines.

Selon d'autres modes de réalisation avantageux du complexe moléculaire, de préférence immunostimulant, de l'invention, le deuxième ligand cible une molécule de surface de CPAg sélectionnée dans le groupe constitué par : les récepteurs lectines de type C, les immunoglobulines membranaires, les récepteurs pour la région constante des immunoglobulines (RFc ou FcR), et les molécules de points de contrôle immunitaire (ICP) et leurs ligands (ICP ligands).

Selon des modes de réalisation avantageux de l'invention, le deuxième ligand cible une molécule de surface exprimée essentiellement sur les cellules NK ou NKT, c'est-à-dire une molécule de surface spécifique des cellules NK ou NKT, de préférence sélectionnée dans le groupe constitué par : les récepteurs NKG2D, NKp30, NKp44, NKp46, NKp80, CD56, CD16, les récepteurs KIR, NKG2A, et les ICP PD-1, CTLA-4, TIM-3, TIGIT, LAG-3 et OX40; de préférence NKp44 (CD336), NKp46 (CD335), NCAM (CD56), CTLA-4 et OX40.

Le deuxième ligand est notamment choisi parmi les saccharides qui lient les récepteurs lectines de type C ; les immunoglobulines et leurs fragments comprenant la région constante qui lient les FcR ; les protéines ou les fragments de protéines qui lient la région Fc et/ou ou Fab des immunoglobulines membranaires, tels que décrits dans la Demande FR 2759296, notamment la protéine A de *S. aureus,* son fragment BB (SEQ ID NO : 1) et son dérivé ZZ (SEQ ID NO : 2), de préférence r ZZ (SEQ ID NO : 2).Alternativement, le deuxième ligand est un anticorps dirigé contre ces molécules de surface de CPAg, de cellule NK ou NKT ou un fragment de cet anticorps contenant au moins le paratope (domaine de liaison à l'Ag), tels que les fragments Fab, Fab', F(ab')₂, Fv ou Fv simple chaîne (scFv pour *single-chain Fv),* Fabc, et fragment Fab comprenant une portion du domaine Fc.

L'anticorps ou le fragment d'anticorps est notamment dirigé contre une molécule de surface spécifique des cellules immunitaires de la réponse innée ou adaptative incluant les CPAg, les cellules NK et NKT, tel que de façon non-limitative PDL1, PDL2, CD155, CD80, CD86, CD40, OX40L, ICOSL (CD275), CD70 ; PDL1, PDL2, CD155, CD80, CD86, CD40, OX40L, ICOSL (CD275), CD70, PD-1, CTLA-4, TIM-3, TIGIT, LAG-3 ; de préférence PDL1 et ICOSL (CD275) ou PDL1, ICOSL (CD275), CTLA-4 et OX40.

**.** L'anticorps ou le fragment d'anticorps peut également être dirigé contre une molécule de surface spécifique des CPAg et en particulier des cellules dendritiques tel qu'un anticorps anti-RFcgamma (I, II et/ou III) ou anti-récepteur lectine de type C, en particulier anti-DEC-205 ou anti-DC-SIGN (CD209) ; anti-DEC-205, anti-DC-SIGN (CD209) ou anti-chaîne invariante li (CD74). L'anticorps peut être un agoniste ou un antagoniste deladite molécule de surface ; par exemple l'anticorps est un agoniste d'une molécule de surface activatrice ou un antagoniste d'une molécule de surface inhibitrice. De préférence, ledit anticorps ou fragment d'anticorps est humain ou humanisé. De tels anticorps sont bien connus de l'Homme du métier et disponibles commercialement.

Selon des modes de réalisation avantageux du complexe moléculaire, de préférence immunostimulant, de l'invention, le deuxième ligand est sélectionné dans le groupe constitué par : (i) les anticorps dirigés contre lesdites molécules de surface des cellules présentatrices d'antigène, des cellules NK ou NKT et leurs fragments contenant au moins le paratope tels que les fragments Fab, Fab', F(ab')₂, Fv, scFv, Fabc et Fab avec au moins une portion du domaine Fc (ii) les immunoglobulines, de préférence les IgG, et leurs fragments comprenant au moins la région Fc, et (iii) les protéines et les fragments de protéines qui lient la région Fc et/ou Fab des anticorps, notamment la protéine A de *S. aureus,* son fragment BB (SEQ ID NO : 1) et son dérivé ZZ (SEQ ID NO : 2).

Les anticorps dirigés contre lesdites molécules de surface des cellules présentatrices d'antigène sont de préférence choisis parmi les anticorps anti-RFcgamma (I, II et/ou III), anti-DC-SIGN (CD209), anti-DEC-205, anti-CD206, anti-CD40; les anticorps anti-RFcgamma (I, II et/ou III), anti-DC-SIGN (CD209), anti-DEC-205, anti-CD206, anti-CD40, anti-chaîne invariante li (CD74), et anti-ICOSL (CD275) ; de manière préférée les anticorps anti-DC-SIGN (CD209) et anti-DEC-205 ou les anticorps anti-DC-SIGN (CD209), anti-DEC-205, anti-chaîne invariante li (CD74), et anti-ICOSL (CD275).

Les anticorps dirigés contre lesdites molécules de surface de cellules NK ou NKT sont de préférence choisis parmi les anticorps anti-CD16, anti-CD56, anti-CD335, anti-CD336, anti-NKp30, anti-NKG2D, anti-NKp80, anti-Ly49H, anti-NKG2A, anti-PD-1, anti-CTLA-4, anti-TIM-3, anti-TIGIT, anti-LAG-3 et anti-OX40 ; de manière préférée les anticorps anti-NCAM (CD56), anti-Nkp46 (CD335), anti-Nkp44 (CD336), anti-CTLA-4 et anti-OX40.

Les ligands supplémentaires du complexe moléculaire, de préférence un complexe immunostimulant, sont avantageusement choisis parmi les ligands de GAG sulfaté et les ligands de molécules de surface de CPAg tels que définis ci-dessus.

Un premier type de complexe immunostimulant préféré selon l'invention consiste en un peptide ligand de GAG sulfaté tel que défini ci-dessus (premier ligand) associé à une protéine ou un fragment de protéine qui lie la région Fc et/ou Fab des immunoglobulines tel que le fragment BB de la protéine A de *Staphylococcus aureus* et son dérivé ZZ (deuxième ligand), de préférence sous la forme d'une protéine de fusion comprenant le premier et le deuxième ligand. Un premier type de complexe particulièrement préféré consiste en une protéine de fusion comprenant un premier ligand choisi parmi Tat49-57 (SEQ ID NO : 3), Tat37-57 (SEQ ID NO : 8), Tat22-57C(22-37)S (SEQ ID NO 9), le domaine R de la toxine diphtérique (DTR : SEQ ID NO : 5) ou le fragment DT453-467 (SEQ ID NO 7) ; et un deuxième ligand choisi parmi le fragment BB de la protéine A (SEQ ID NO : 1) ou son dérivé ZZ (SEQ ID NO : 2) ; de préférence son dérivé ZZ (SEQ ID NO : 2). Le premier et le deuxième ligand sont fusionnés, soit directement, soit par l'intermédiaire d'un peptide espaceur approprié. Dans ce premier type de complexe particulièrement préféré, le deuxième ligand est de préférence en N-terminal et le premier ligand en C-terminal de la protéine de fusion ; le premier et le deuxième ligand sont séparés par un peptide espaceur approprié, notamment choisi parmi SEQ ID NO : 21 à 23. De manière plus préféré, le dit complexe comprend l'une des séquences SEQ ID NO : 12, 14, 16, 18 et 20. Lorsque le complexe comprend le domaine R de la toxine diphtérique (DTR : SEQ ID NO : 5), alors le domaine est avantageusement produit en cellules de mammifères, en particulier sous forme d'une protéine de fusion avec le deuxième ligand, de façon à former des oligomères.

Un deuxième type de complexe immunostimulant préféré selon l'invention consiste en: (i) un peptide ligand de GAG sulfaté tel que défini ci-dessus (premier ligand) lié de façon covalente à un élément de liaison aux immunoglobulines tel que défini ci-dessus, en particulier une protéine ou un fragment de protéine lie la région Fc et/ou Fab des Ac, de préférence uniquement la région Fab, tel que la protéine A de *Staphylococcus aureus* et son fragment BB, de préférence sous la forme d'une protéine de fusion comprenant le premier ligand et l'élément de liaison aux immunoglobulines, ledit premier ligand associé de façon covalente à l'élément de liaison aux immunoglobulines étant complexé à (ii) une immunoglobuline, de préférence une IgG, ou un fragment comprenant au moins la région Fc (deuxième ligand). De préférence, une immunoglobuline entière, de manière préférée une IgG entière. Le premier ligand et l'élément de liaison aux immunoglobulines sont fusionnés, soit directement, soit par l'intermédiaire d'un par l'intermédiaire d'un peptide espaceur approprié. Un deuxième type de complexe particulièrement préféré consiste en une protéine de fusion comprenant un premier ligand choisi parmi Tat49-57 (SEQ ID NO : 3), Tat 37-57 (SEQ ID NO : 8), Tat22-57C(22-37)S (SEQ ID NO 9), le domaine R de la toxine diphtérique (DTR : SEQ ID NO : 5) ou le fragment DT453-467 (SEQ ID NO 7) et un élément de liaison aux immunoglobulines comprenant le fragment BB de la protéine A (SEQ ID NO : 1), ladite protéine de fusion étant complexée à une immunoglobuline entière. Dans ce deuxième type de complexe particulièrement préféré, l'élément de liaison aux immunoglobulines (BB) est de préférence en N-terminal et le premier ligand en C-terminal de la protéine de fusion ; l'élément de liaison aux immunoglobulines (BB) et le premier ligand sont séparés par un peptide espaceur approprié, notamment choisi parmi SEQ ID NO : 21 à 23. De manière plus préféré, le dit complexe comprend l'une des séquences SEQ ID NO : 16 ou 18. Lorsque le complexe comprend le domaine R de la toxine diphtérique (DTR : SEQ ID NO : 5), alors le domaine est avantageusement produit en cellules de mammifères, en particulier sous forme d'une protéine de fusion avec l'élément de liaisons aux immunoglobulines, de façon à former des oligomères.

Un troisième type de complexe immunostimulant préféré selon l'invention consiste en : un peptide ligand de GAG sulfaté tel que défini ci-dessus (premier ligand) associé à un Ac sélectionné dans le groupe constitué par un Ac anti-RFcgamma (I, II, et/ou III), un Ac anti-DEC-205, un Ac anti-DC-SIGN (CD209), un Ac anti-chaîne invariante li (CD74), un Ac anti- ICOSL (CD275), un Ac anti- NKp46 (CD335), un Ac anti- NKp44 (CD336), un Ac anti-NCAM (CD56), un Ac anti-CTLA-4, un Ac anti-PDL1, un Ac anti-OX40, et un fragment des Ac précédents comprenant au moins le paratope. De préférence, le peptide ligand de GAG sulfaté tel que défini ci-dessus (premier ligand) est lié de façon covalente à un élément de liaison aux immunoglobulines tel que défini ci-dessus, en particulier une protéine ou un fragment de protéine qui lie la région Fc et/ou Fab des immunoglobulines tel que le fragment BB de la protéine A de *Staphylococcus aureus* et son dérivé ZZ, de préférence sous la forme d'une protéine de fusion du premier ligand avec l'élément de liaison aux immunoglobulines. Le premier ligand et l'élément de liaison aux immunoglobulines sont fusionnés, soit directement, soit par l'intermédiaire d'un par l'intermédiaire d'un peptide espaceur approprié. Un troisième type de complexe particulièrement préféré consiste en une protéine de fusion comprenant un premier ligand choisi parmi Tat49-57 (SEQ ID NO : 3), Tat37-57 (SEQ ID NO : 8), Tat22-57C(22-37)S (SEQ ID NO 9), le domaine R de la toxine diphtérique (DTR : SEQ ID NO : 5), ou le fragment DT453-467 (SEQ ID NO 7) et un élément de liaison aux immunoglobulines comprenant le fragment BB de la protéine A (SEQ ID NO : 1) ou son dérivé ZZ (SEQ ID NO : 2), ladite protéine de fusion étant complexée à un anticorps anti-RFcgamma (I, II, et/ou III), anti-DEC-205, anti-DC-SIGN, anti-CD74, anti-CD275, anti-CD335, anti-CD336, anti-CD56, anti-CTLA-4, anti-PDL1, anti-OX40 ou à un fragment des anticorps précédents comprenant au moins le paratope. Dans ce troisième type de complexe particulièrement préféré, l'élément de liaison aux immunoglobulines (BB ou ZZ) est de préférence en N-terminal et le premier ligand en C-terminal de la protéine de fusion ; l'élément de liaison aux immunoglobulines (BB ou ZZ) et le premier ligand sont séparés par un peptide espaceur approprié, notamment choisi parmi SEQ ID NO : 21 à 23. De manière plus préféré, le dit complexe comprend l'une des séquences SEQ ID NO : 12, 14, 16, 18 ou 20. Lorsque le complexe comprend le domaine R de la toxine diphtérique (DTR : SEQ ID NO : 5), alors le domaine est avantageusement produit en cellules de mammifères, en particulier sous forme d'une protéine de fusion avec l'élément de liaisons aux immunoglobulines, de façon à former des oligomères. De manière plus préféré, le dit complexe comprend un anticorps anti-DEC-205, anti-DC-SIGN, anti-CD74, anti-CD275, anti-CD335, anti-CD336, anti-CD56, anti-CTLA-4, anti-PDL1 ou anti-OX40

Selon des modes de réalisation préférés de l'invention, ledit complexe est utilisé en tant qu'immunostimulant, de préférence pour activer des cellules présentatrices de l'antigène, notamment des cellules dendritiques oudes monocytes, pour activer des cellules NK ou NKT, et/ou pour activer la sécrétion des cytokines IL-6 et/ou IL-12.

L'invention a en outre pour objet une composition immunomodulatrice, de préférence immunostimulante, comprenant au moins un complexe immunomodulateur, de préférence immunostimulant selon l'invention, et au moins un véhicule pharmaceutiquement acceptable, une substance porteuse et/ou un adjuvant.

Les véhicules pharmaceutiquement acceptables sont ceux classiquement utilisés.

Les adjuvants sont les adjuvants de l'immunité humorale et/ou cellulaire classiquement utilisés en immunothérapie. Les adjuvants sont avantageusement choisis dans le groupe constitué par : des émulsions huileuses, des substances minérales, des extraits bactériens, la saponine, l'hydroxyde d'alumine, le monophosphoryl -lipide A, le squalène et les ligands de TLR, notamment les oligodéoxynucléotides comprenant au moins une séquence CpG (oligodéoxynucléotide CpG) qui sont des ligands de TLR9, ou l'acide polyinosinique-polycytidylique (poly(I)-poly(C) ou poly I-C) qui est un ligand de TLR3. Selon des modes de réalisation préférés de l'invention, ladite composition comprend au moins un adjuvant, de préférence un oligodéoxynucléotide CpG, de l'acide polyinosinique-polycytidylique ou un mélange d'oligodéoxynucléotide(s) CpG, et d'acide polyinosinique-polycytidylique.

**Les** substances porteuses sont celles classiquement utilisées. Il s'agit notamment de liposomes unilamellaires ou multilamellaires, d'ISCOMS, de virosomes (*virus-like particles*), de micelles de saponine, de microsphères solides de nature saccharidique (poly(lactide-co-glycolide)) ou aurifère, et de nanoparticules. Selon des modes de réalisation préférés de l'invention, ladite composition comprend au moins une substance porteuse, par exemple une nanoparticule ou un mélange de nanoparticules.

La composition immunomodulatrice, de préférence immunostimulante, selon l'invention comprend un complexe comprenant 2 ligands ou plus ou un mélange de complexes différents, le ou lesdits complexes étant éventuellement liés entre eux par des liaisons covalentes ou non-covalentes et/ou incorporés à l'intérieur ou à la surface d'une particule telle qu'un liposome, un virosome ou une nanoparticule.

Selon des modes de réalisation particuliers de l'invention, la composition comprend un polynucléotide ou un mélange de polynucléotides codant pour des ligands de CPAg, NK ou NKT qui sont des protéines, polypeptides ou peptides. Le polynucléotide consiste en un acide nucléique recombinant, synthétique ou semi-synthétique qui est exprimable dans des cellules de l'hôte auquel la composition est administrée. L'acide nucléique peut être un ADN, un ARN, notamment un ARNm, un acide nucléique mixte (ADN/ARN) et peut être modifiée. Par exemple, ladite composition comprend un polynucléotide comprenant une séquence codant pour une protéine de fusion comprenant au moins les séquences codantes du premier et du deuxième ligand, fusionnées en phase de façon appropriée, et éventuellement une séquence codant pour un élément de liaison tel que défini ci-dessus. Alternativement, la composition comprend un mélange de polynucléotides comprenant au moins un premier polynucléotide comprenant une séquence codant pour le premier ligand et un second polynucléotide comprenant une séquence codant pour le deuxième ligand, ledit premier ou second nucléotide comprenant également une séquence codant pour un élément de liaison tel que défini ci-dessus. Le ou lesdits polynucléotides sont de préférence insérés dans un ou plusieurs vecteur d'expression comprenant des séquences régulatrices de la transcription et/ou de la traduction appropriées (promoteur, activateur de transcription, terminateur de transcription, signal de polyadénylation) pour l'expression du premier ligand et du deuxième ligand, et éventuellement des autres ligands *in* vivo chez les individus auxquels la composition est administrée. De nombreux vecteurs utilisables en thérapie sont connus en eux-mêmes. On peut utiliser entre autres, des vecteurs viraux (adénovirus, rétrovirus, lentivirus, AAV) et des vecteurs non-viraux (ADN-nu), notamment un plasmide, dans lesquels a été insérée préalablement la séquence d'intérêt. Alternativement, le ou lesdits polynucléotides sont des ARNm, de préférence modifiés. L'utilisation d'ARNm en thérapie est bien connue de l'Homme du métier (revue par exemple Drew Weissman, Expert Reviews of Vaccines, October 2014, 1-17, doi : 10.1586/14760584.2015.973859).

Selon des modes de réalisation particuliers de l'invention, la composition comprend des cellules modifiées par la composition selon l'invention. Par exemple, les cellules sont modifiées par un polynucléotide, un mélange de polynucléotides ou un vecteur tel que défini ci-dessus ou chargées avec un complexe de ligands tel que défini ci-dessus. La cellule est notamment une cellule présentatrice d'antigène naturelle telle qu'une cellule dendritique ou une cellule présentatrice de l'antigène artificielle, telle que des exosomes dérivés de cellules dendritiques ou des vésicules dérivées de cellules exprimant les ligands du complexe moléculaire selon l'invention. Par exemple, les cellules sont des cellules présentatrices d'antigène d'un individu à traiter, notamment des cellules dendritiques qui sont modifiées ex *vivo* avant d'être ré-administrées à l'individu (Thérapie cellulaire *ex vivo*).

La composition immunomodulatrice peut comprendre en outre au moins un autre agent thérapeutique, notamment un agent anticancéreux, anti-infectieux, un autre agent immunomodulateur ou un antigène vaccinal spécifique de la maladie à traiter, ledit antigène vaccinale étant avantageusement associé à une substance porteuse ou inclus dans un vecteur approprié. Selon des modes de réalisation préférés de l'invention, ladite composition comprend en outre au moins un inhibiteur de point de contrôle immunitaire (Marin-Acevedo J. et al., 2018, Hematol. Oncol., 11:39) tel que de façon non-limitative un anti-PD-1, un anti-PDL-1 ou un anti-CTLA4, notamment un anticorps, de préférence monoclonal, dirigé contre la molécule PD-1, PDL-1 ou CTLA4, de préférence la molécule humaine hPD-1, hPDL-1 ou hCTLA4. La composition selon l'invention comprend avantageusement un anti-PD-1, en particulier un anticorps monoclonal anti-PD-1, de préférence anti-hPD-1. Selon d'autres modes de réalisation préférés de l'invention, ladite composition comprend en outre, au moins un antigène vaccinal spécifique de la maladie à traiter, de préférence associé à une substance porteuse ou inclus dans un vecteur approprié.

La composition immunomodulatrice, de préférence immunostimulante, comprend une dose efficace de complexe(s), polynucléotide(s), vecteur(s), cellule(s) suffisante pour induire une réponse immunitaire capable de produire un effet thérapeutique sur la maladie à traiter, c'est-à-dire de diminuer les symptômes de cette maladie. Il s'agit notamment de l'atténuation des conséquences de l'action par un pathogène (infectieux ou non-infectieux) ou bien de la réduction la croissance d'une tumeur, chez un individu traité avec cette composition. Cette dose est déterminée et ajustée en fonction de facteurs tels que l'âge, le sexe et le poids du sujet. La composition immunomodulatrice, de préférence immunostimulante, selon l'invention se présente sous une forme galénique adaptée l'administration choisie. La composition est généralement administrée selon les protocoles usuels d'immunothérapie à des doses et pendant une durée suffisante pour induire une réponse immunitaire efficace contre la pathologie à traiter. L'administration peut être intratumorale, sous-cutanée, intramusculaire, intraveineuse, intradermique, intrapéritonéale, orale, sublinguale, rectale, vaginale, intra-nasale, par inhalation ou par application transdermique. La composition se présente sous une forme galénique adaptée à une administration choisie.

Les polynucléotides isolés ou insérés dans un vecteur plasmidique sont introduits dans l'individu à soigner, soit en utilisant des méthodes physiques telles que l'électroporation, soit en les associant à toute(s) substance(s) permettant le passage de la membrane plasmique, tels que des transporteurs comme les nanotransporteurs, des liposomes, des lipides ou des polymères cationiques. En outre, on peut avantageusement combiner ces méthodes, par exemple en utilisant l'électroporation associée à des liposomes.

La composition immunomodulatrice, de préférence immunostimulante, selon la présente invention est utilisée en immunothérapie, en particulier antitumorale ou anti-infectieuse. La composition immunomodulatrice, de préférence immunostimulante, selon la présente invention est utilisée à titre préventif ou curatif, c'est-à-dire pour prévenir une pathologie chez des invividus ou traiter des individus souffrant d'une pathologie. Selon des modes de réalisation préférés de l'invention, ladite composition est utilisée à titre curatif, c'est-à-dire pour traiter des individus souffrant d'une pathologie Elle peut être utilisée en combinaison avec d'autres traitements, thérapeutique ou chirurgical, notamment en combinaison avec d'autres agents thérapeutiques tels que définis ci-dessus, la composition selon l'invention et les autres agents thérapeutiques pouvant être administrés de façon simultanée, séparée ou séquentielle.

Selon des modes de réalisation préférés de l'invention, ladite composition est utilisée dans le traitement du cancer. Les cancers sont tout type de cancer pouvant bénéficier de l'immunothérapie tel que de façon non limitative: les cancers du sein, du colon, de la prostate, de l'œsophage, de l'estomac, des poumons, les cancers ORL (oto-rhino-laryngé), de la peau, des ovaires, de l'utérus, du cerveau, du foie, des reins.

Selon d'autres modes de réalisation préférés de l'invention, ladite composition est utilisée dans le traitement des maladies infectieuses, notamment celles pour lesquelles les agents infectieux persistent dans l'organisme. Les maladies infectieuses sont tout type de maladie infectieuse pouvant bénéficier de l'immunothérapie (Wykes MN et al., Nat. Rev. Immunol., 2018, 18:91-104) telle que de façon non limitative : les infections virales, bactériennes, fongiques ou parasitaires, notamment les infections par le VIH, le VHB, le VHC, mycobaterium tuberculosis ou plasmodium falciparum. Lorsque ledit complexe comprend Tat ou un fragment de Tat tels que définis ci-dessus, alors ladite composition est utilisée dans le traitement de maladies infectieuses autres que l'infection par le VIH (SIDA). Lorsque ledit complexe comprend DTR ou un fragment de DTR tels que définis ci-dessus, alors ladite composition est utilisée dans le traitement de maladies infectieuses autres que la diphtérie (infection par le bacille diphtérique, *Corynebacterium diphteriae).*

La composition immunomodulatrice, de préférence immunostimulante, selon la présente invention est utilisée, soit en thérapie classique, soit en thérapie cellulaire, ou bien encore par une combinaison des deux approches.

La thérapie cellulaire comprend la préparation de cellules présentatrices d'antigènes, notamment des cellules dendritiques, ou des cellules NK ou NKT, par un protocole classique comprenant l'isolement de cellules mononucléées du sang périphérique (PBMC) d'un patient à traiter et la mise en culture des cellules dendritiques, NK ou NKT, en présence de complexe(s) moléculaires(s), polynucléotide(s), vecteur(s) tels que définis ci-dessus. Dans une seconde étape les cellules présentatrices d'antigène, NK ou NKT chargées avec le ou lesdits complexe(s) moléculaires(s) ou modifiées par ledit ou lesdits polynucléotide(s) ou vecteur(s) sont réinjectées au patient.

La présente invention a également pour objet une méthode d'immunothérapie, en particulier anti-tumorale ou anti-infectieuse, caractérisée en ce qu'elle comprend l'administration d'une composition immunomodulatrice, de préférence immunostimulante telle que définie ci-dessus, à un individu, par tout moyen approprié tel que défini ci-dessus.

La présente invention a également pour objet l'utilisation d'une composition immunomodulatrice, de préférence immunostimulante telle que définie ci-dessus pour la préparation d'un médicament destiné à l'immunothérapie, de préférence anti-tumorale ou anti-infectieuse.

Les complexes de ligands de CPAg, de cellules NK ou NKT selon l'invention sont préparés par les techniques classiques connues de l'Homme du métier, à savoir :
- les ligands des molécules de surface des CPAgs, des cellules NK ou NKT peuvent être produits par synthèse chimique ou par expression d'un ADN recombinant dans un système cellulaire approprié, eucaryote ou procaryote. Les peptides et protéines peuvent être synthétisés en phase solide, selon la technique Fmoc, originellement décrite par Merrifield et al. (J. Am. Chem. Soc., 1964, 85, 2149-) et purifiés par chromatographie liquide haute performance en phase inverse. Les polypeptides et les protéines peuvent être produits à partir des ADNc correspondants, clonés dans un vecteur d'expression eucaryote ou procaryote approprié, les polypeptides ou protéines produits dans les cellules modifiées par le vecteur recombinant sont purifiées par tout moyen approprié, notamment par chromatographie d'affinité. Des Ac dirigés contre des molécules de surface de CPAgs, de cellules NK ou NKT sont bien connus et disponibles commercialement. Par exemple, à titre d'exemple non-limitatif, les Ac anti-CD205 (#555831) ; anti-CD206 (#555952) ; anti-CD209 (#551186) ; anti-HLA-DR (#555556) sont disponibles chez BECTON-DICKINSON, alors que l'Ac anti-CD56 est disponible chez Biolegend (#304622) et les Ac anti-CD335 (AM31284AF-N) et anti-CD336 (#AM50346PU-N) sont disponibles chez Origene. Alternativement, des Ac monoclonaux peuvent être produits par les techniques classiques connues de l'homme du métier. Par exemple, les Ac monoclonaux sont produits à partir d'hybridomes obtenus par fusion de lymphocytes B d'un animal immunisé par la molécule de surface de CPAgs avec des myélomes, selon la technique de Köhler et Milstein (Nature, 1975, 256, 495-497) ; les hybridomes sont cultivés *in vitro,* notamment dans des fermenteurs ou produits in vivo, sous forme d'ascite ; alternativement lesdits Ac monoclonaux sont produits par génie génétique comme décrit dans le brevet américain US 4,816,567. Les Ac humanisés sont produits par des méthodes générales comme celles décrites dans la Demande Internationale WO 98/45332. Les fragments d'Ac sont produits à partir des régions V_{H} et V_{L} clonées, à partir des ARNm d'hybridomes ou de lymphocytes spléniques d'un mammifère immunisé; par exemple, les fragments Fv, scFv ou Fab sont exprimés à la surface de phages filamenteux selon la technique de Winter et Milstein (Nature, 1991, 349, 293-299); après plusieurs étapes de sélection, les fragments d'Ac spécifiques de l'Ag sont isolés et exprimés dans un système d'expression approprié, par les techniques classiques de clonage et d'expression d'ADN recombinant. Les Ac ou leurs fragments tels que définis ci-dessus, sont purifiés par les techniques classiques connues de l'Homme du métier, telles que la chromatographie d'affinité.
- l'association covalente du premier ligand (L1) au deuxième ligand (L2) d'une molécule de surface des CPAgs ou des cellules NK ou NKT peut-être réalisé par la construction d'une protéine de fusion dans laquelle les séquences nucléotidiques codant pour L1, et L2 sont fusionnées en phase, dans l'ordre approprié, soit directement, soit par l'intermédiaire d'une séquence nucléotidique codant pour peptide espaceur approprié. En fonction des tailles respectives des séquences en acides aminés de L1 et L2, elles sont soit fusionnées au niveau de leurs extrémités (extrémité N-terminale de l'une des séquences fusionnée à l'extrémité C-terminale de l'autre séquence) ou l'une des séquences est insérée dans l'autre séquence à un site approprié qui n'a pas d'effet délétère sur la liaison du ou des ligands à son récepteur exprimé à la surface des CPAg ou des cellules NK ou NKT. Alternativement, le(s) ligand(s) peuvent être couplés de façon covalente, par tout moyen approprié. Le couplage est effectué par l'intermédiaire de groupements réactionnels initialement présents ou préalablement introduits sur le(s) ligand(s). Le couplage peut notamment être réalisé au niveau de résidus d'acides aminés dont la chaîne latérale comprend une fonction réactive. Parmi ces acides aminés, on peut citer les acides aminés polaires comprenant une fonction : -OH [sérine (S), thréonine (T) ou tyrosine (Y)], -SH [cystéine (C)], -NH₂ [lysine (K) ou arginine (R)], -COOH [acide aspartique (D) ou acide glutamique (E)], et les acides aminés polaires à chaîne latérale fonctionnalisée par addition d'une fonction réactive, notamment un chloro- ou bromo-acétyl réactif avec les groupements thiol ou un groupement hydrazine réactif avec les aldéhydes. Le ligand est couplé par tout moyen approprié ; ces moyens qui sont connus de l'Homme du métier incluent notamment le couplage à l'aide de réactifs homobifonctionnels comme le glutaraldéhyde ou le dithiobis-(succinimidyl propionate). De préférence, le couplage est réalisé à l'aide de réactifs hétérobifonctionnels, notamment le m-maleimidobenzoyl-N-hydroxysuccinimide (SMCC) ou le sulfo-SMCC qui contiennent chacun un groupement maléimide capable de réagir avec les thiols libres. Dans ce cas, le SMCC est préalablement lié de façon covalente à une fonction amine présente sur le ligand. Dans le même temps, un autre réactif hétérobifonctionnel (comme le N-succinimidyl S-acetylthioacétate qui contient un groupement thio-ester clivable à l'hydroxylamine ou le succinimidyl-pyridyl-dithiopropionate, qui contient un pont disulfure réductible en conditions douce), est associé à une fonction amine du second partenaire qui est un des ligands. Le second partenaire est ensuite traité à l'hydroxylamine ou avec un réducteur pour permettre la libération du thiol. Le composé thiolé est alors incubé avec le composé ayant incorporé le maléimide et le couplage est obtenu par réaction du groupement thiol sur le groupement maléimide. Ce type de couplage covalent est notamment décrit dans Léonetti et al., J. Exp. Med., 1999, 189, 1217-1228. Il est aussi possible de libérer un groupement thiol déjà présent sur un des composés pour ensuite réaliser son couplage à un autre composé qui a été préalablement modifié à l'aide de SMCC. Cette méthode, qui est souvent employée pour coupler des Ac à des ligands, est notamment décrite dans Ishikawa et al., J. Immunoassay, 1983, 4, 209-327.
- les complexes non-covalents sont préparés par mise en contact du deuxième ligand (L2) avec le premier ligand (L1) dans des conditions permettant aux deux partenaires d'interagir. Cette interaction peut impliquer un élément de liaison, notamment une protéine ou un peptide, qui possède une affinité élevée et spécifique, pour l'un des partenaires du complexe (L1 ou L2). En particulier, l'affinité de l'élément de liaison pour ce partenaire, dans le complexe, est suffisante pour qu'il ne se dissocie pas immédiatement de ce complexe *in vivo.* Lorsque l'un des ligands est une immunoglobuline, l'élément de liaison est un élément de liaison aux immunoglobulines tel que décrit dans la Demande FR 2759296. Par exemple, un élément de liaison aux immunoglobulines est lié de façon covalente à L1, de façon à former un complexe non-covalent avec L2.
- les polynucléotides selon l'invention sont obtenus par les méthodes classiques, connues en elles-mêmes, en suivant les protocoles standards connus de l'homme du métier. Par exemple, ils peuvent être obtenus par amplification d'une séquence nucléique par PCR ou RT-PCR, par criblage de banques d'ADN génomique par hybridation avec une sonde homologue, ou bien par synthèse chimique totale ou partielle. Les vecteurs recombinants sont construits et introduits dans des cellules hôtes par les méthodes classiques d'ADN recombinant et de génie génétique, qui sont connues en elles-mêmes.

La mise en œuvre de l'invention utilise, sauf indication contraire, des méthodes classiques d'immunologie, de culture cellulaire, de biologie cellulaire, de biologie moléculaire et d'ADN recombinant qui sont connues de l'homme du métier.

### Brève description des dessins

D'autres caractéristiques, détails et avantages de l'invention apparaîtront à la lecture de la description détaillée ci-après qui se réfère à des exemples de mise en œuvre de la présente invention, et à l'analyse des dessins annexés, sur lesquels :
**Fig. 1**

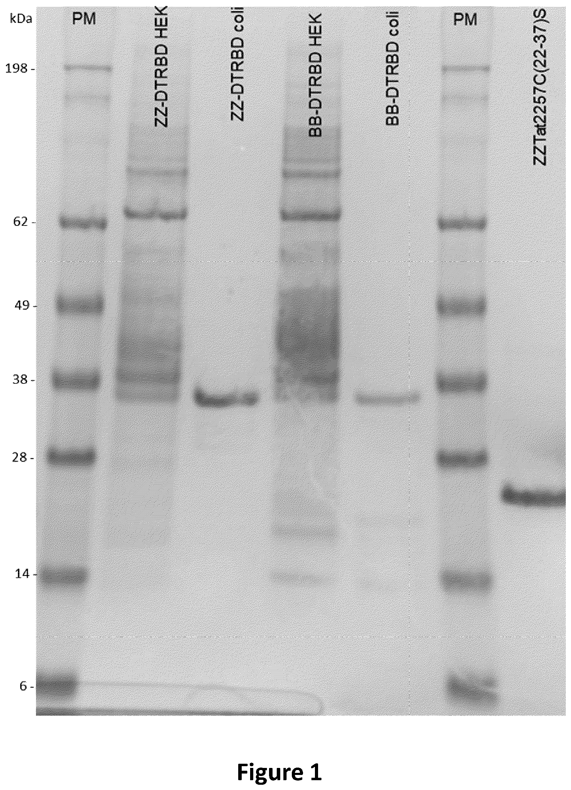

   [Fig. 1] montre la masse moléculaire et le degré d'homogénéité de complexes moléculaires produits chez *E. coli* ou en cellules HEK. Les protéines ZZ-DTRBD_{HEK}, ZZ-DTRBD_{coli},, BB-DTRBD_{HEK}, BB-DTRBD_{coli} et ZZ-Tat_{22-57C(22-37)S} ont été analysées par électrophorèse en conditions dénaturantes. Les protéines ont été déposées en présence de marqueurs de poids moléculaires. Après migration électrophorétique les protéines ont été colorées au bleu de coomassie.
**Fig. 2**

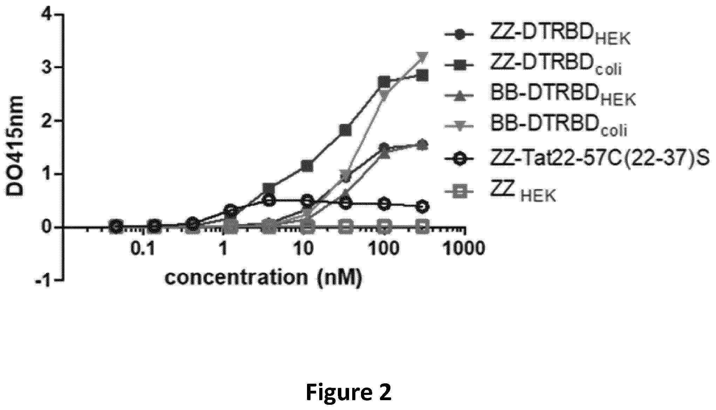

   [Fig. 2] montre la liaison de différents complexes moléculaires à l'héparine. Des séries de dilutions des protéines ZZ libre, ZZ-DTRBD_{HEK}, ZZ-DTRBD_{coli}, BB-DTRBD_{HEK}, ZZ-DTRBD_{coli} et ZZ-Tat_{22-57C(22-37)S} ont respectivement été incubées à pH7,2 dans des puits plaques de microtitration préalablement adsorbées avec des IgG de lapin. Après 4 heures les plaques ont été lavées et de l'héparine-biotinylée a été ajouté. Après 30 minutes d'incubation la liaison de l'héparine aux plaques a été détectée à l'aide de streptavidine couplée à la péroxydase et d'un substrat de cet enzyme (ABTS). La liaison à l'héparine est considérée significative quand le signal de densité optique s'avère au moins égal à 50% du signal mesuré quand ZZ-Tat_{22-57C(22-37)S} est incubé à 100nM pH7,2 sur les plaques de microtitration.
**Fig. 3**

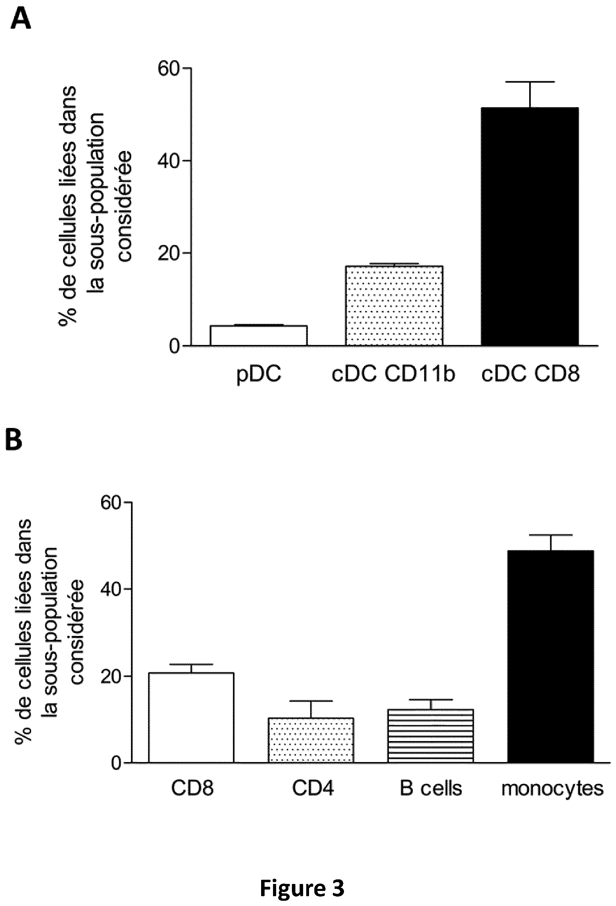

   [Fig. 3] montre les types cellulaires liés par la protéine de fusion ZZ-DTRBD_{HEK} dans une population de splénocytes de souris. Des splénocytes ont été incubés en présence ou en absence d'une quantité fixe de ZZ-DTRBD_{HEK} (100nM). Une série d'Ac fluorescents spécifiques des pDC, cDC CD11b+ et cDC CD8+ **(A) ;** lymphocytes T CD4+, T CD8+, lymphocytes B, monocytes **(B)** a ensuite été ajoutée. Après 30 minutes à 4 °C, les cellules ont été lavées et analysées par cytométrie de flux. Le pourcentage de cellules liées par ZZ-DTRBD_{HEK} dans la sous-population considérée est représenté.
**Fig. 4**

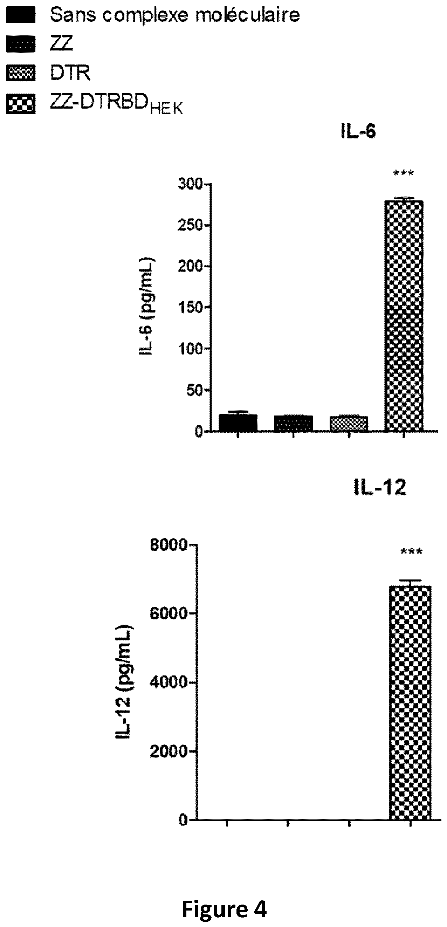

   [Fig. 4] montre que ZZ-DTRBD_{HEK} induit la sécrétion d'IL-6 et d'IL-12 par des splénocytes. Une quantité fixe de ZZ, DTRBD ou ZZ-DTRBD_{HEK} (1µM) a été incubée avec des splénocytes de souris. Après 24h les surnageants ont été prélevés et la présence d'IL-6 et IL-12 a été évaluée par dosage immunoenzymatique.
**Fig. 5**

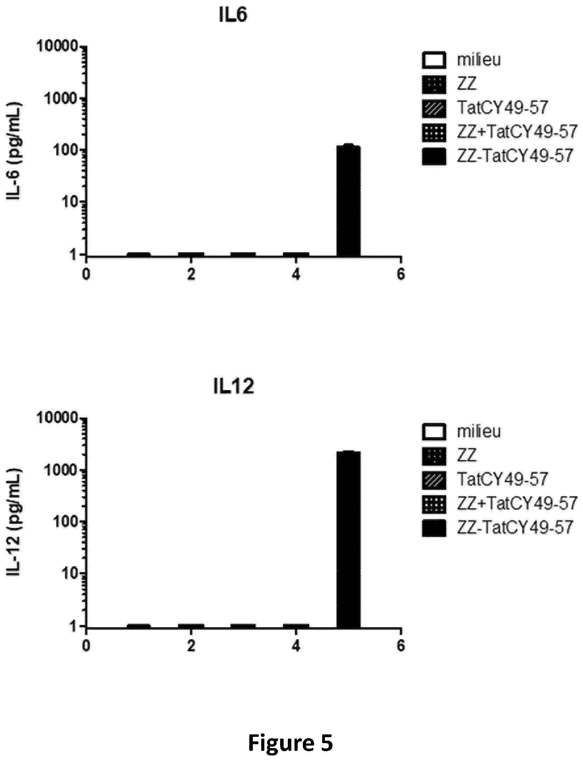

   [Fig. 5] montre que ZZ-Tat_{22-57C(22-37)S} induit la sécrétion d'IL-6 et d'IL-12 par des splénocytes. Une quantité fixe de ZZ, Tat_{CY49-57}, ZZ + Tat_{CY49-57} ou ZZ-Tat_{22-57C(22-37)S} (1µM) a été incubée avec des splénocytes de souris. Après 24h les surnageants ont été prélevés et la présence d'IL-6 et IL-12 a été évaluée par dosage immunoenzymatique.
**Fig. 6**

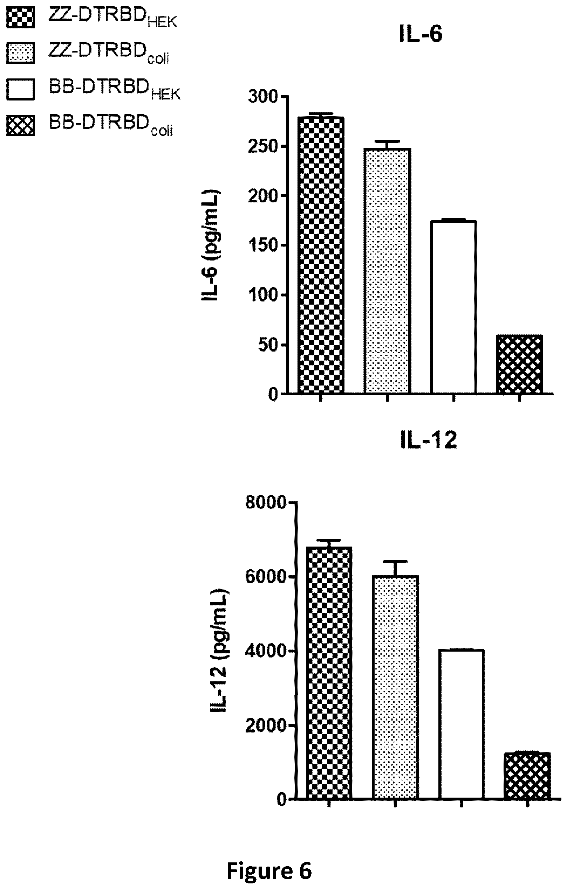

   [Fig. 6] montre que les protéines ZZ-DTRBD_{HEK}, BB-DTRBD_{HEK}, induisent la sécrétion de plus grandes quantités d'IL-6 et d'IL-12 par des splénocytes que ZZ-DTRBD_{coli} et BB-DTRBD_{coli}. Une quantité fixe de ZZ-DTRBD_{HEK}, ZZ-DTRBD_{coli}, BB-DTRBD_{HEK} et BB-DTRBD_{coli} (1µM) a été incubée avec des splénocytes de souris. Après 24h les surnageants ont été prélevés et la présence d'IL-6 et IL-12 a été évaluée par dosage immunoenzymatique.
**Fig. 7**

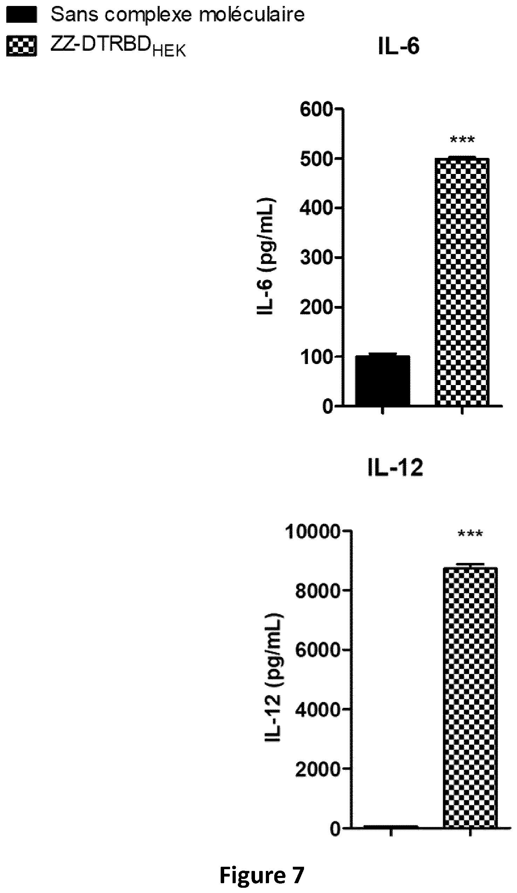

   [Fig. 7] montre que ZZ-DTRBD_{HEK} induit la sécrétion d'IL-6 et d'IL-12 par des cellules dendritiques murines isolées. Des cellules dendritiques ont été isolées par tri magnétique. Les cellules dendritiques ont ensuite été incubées en présence ou en absence d'une quantité fixe de ZZ-DTRBD_{HEK}, (0,6µM). Après 24h, les surnageants ont été prélevés et la présence d'IL-6 et IL-12 a été évaluée par dosage immunoenzymatique.
**Fig. 8**

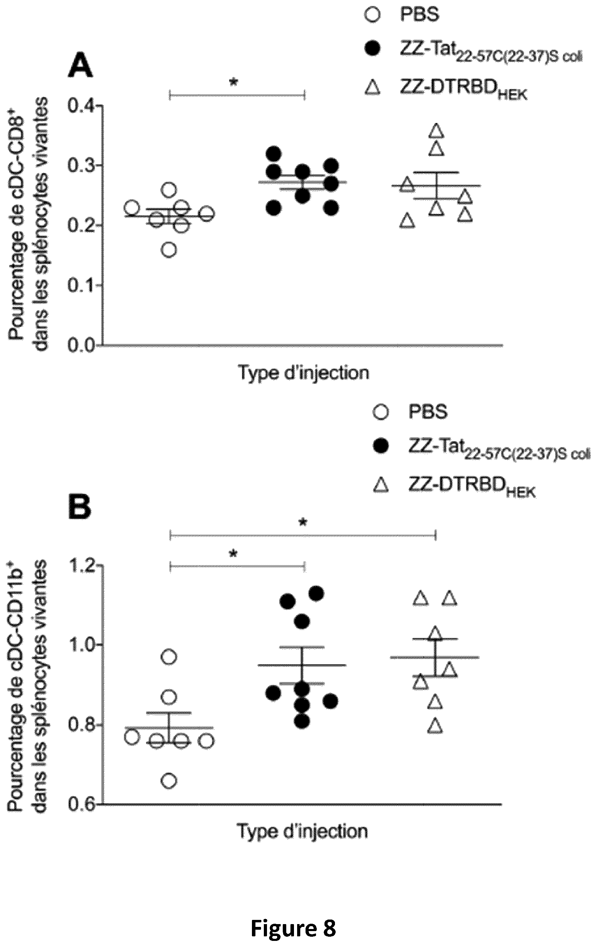

   [Fig. 8A-B] montre que les protéines ZZ-DTRBD_{HEK} et ZZ-Tat_{22-57C(22-37)S} sont capables d'induire l'expansion de cellules dendritiques murines chez la souris. Trois groupes de souris ont été injectés trois fois à trois jours d'intervalle avec un tampon PBS en absence ou en présence d'une quantité fixe de ZZ-DTRBD_{HEK} (5 nmoles par souris) ou ZZ-Tat_{22-57C(22-37)S} (10 nmoles par souris). 24h après la dernière injection les animaux ont été euthanasiés, les rates ont été prélevées et les splénocytes ont été marqués à l'aide d'un cocktail d'Ac fluorescents permettant de distinguer les cDC-CD8+ (CD11c^{high}B220⁻CD8⁺CD11b⁻) **(A),** cDC-CD11b+ (CD11chighB220⁻CD8⁻CD11b⁺) **(B)** et les pDC (CD11c^{int} CD317⁺CD11b⁺) .

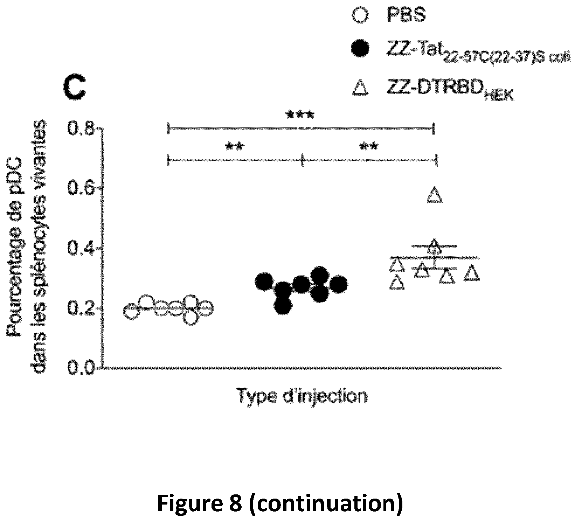

   [Fig. 8C] montre les cellules analysées par cytométrie de flux. Le nombre de cellules positives a été représenté en pourcentage par rapport au nombre total de splénocytes vivants. Les analyses statistiques ont été effectuées par un test de kruskall-wallis (* p<0.05).
**Fig. 9**

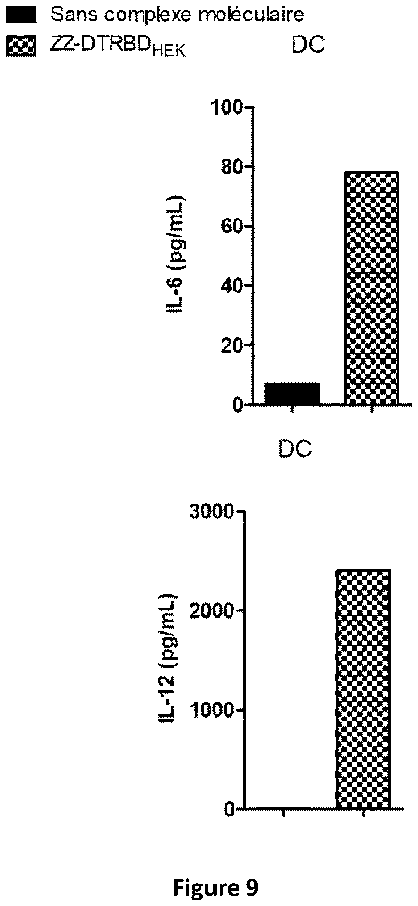

   [Fig. 9] montre que ZZ-DTRBD_{HEK} induit *in vitro* la sécrétion d'IL-6 et d'IL-12 par des DC humaines isolées. Des DC isolées issus de donneurs humains sains ont été incubées en présence d'une quantité fixe de ZZ-DTRBD_{HEK} (1µM). Après 24h, les surnageants sont récoltés pour évaluer la présence des cytokines IL-6 et IL-12 par dosage immunoenzymatique.
**Fig. 10**

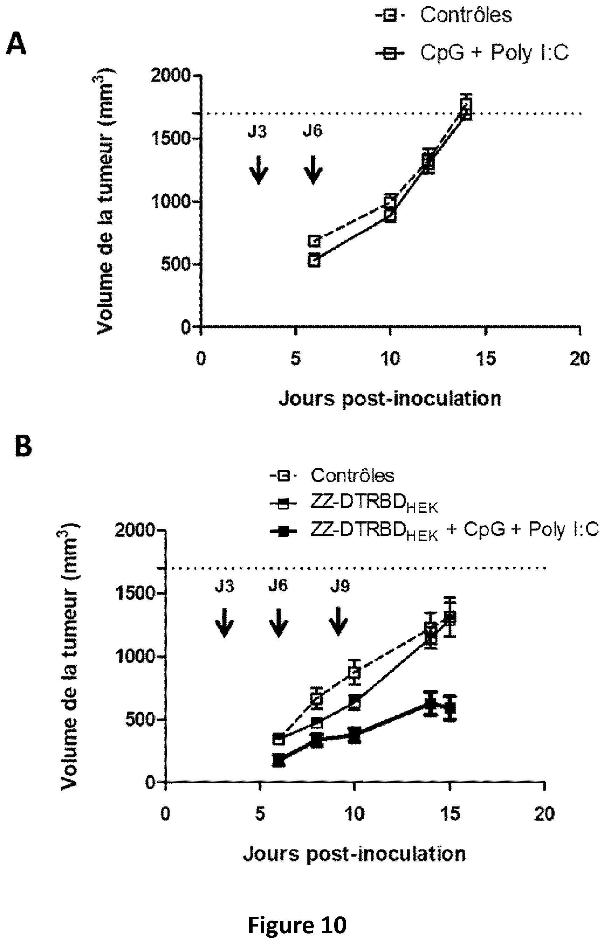

   [Fig. 10] montre que des souris injectées avec des cellules de cancer colorectal présentent une croissance tumorale ralentie lorsqu'elles sont ultérieurement injectées avec ZZ-DTRBD_{HEK} seul ou en association avec un mélange adjuvant. **A** : Seize souris C57BL/6 ont été injectées avec 0.5M de cellules MC38 en sous-cutané. Trois jours plus tard, huit souris ont été injectées avec le mélange adjuvant CpG-B 1018/Poly I:C. (30 µg pour chaque adjuvant) et huit ont été non injectées (Contrôles). Une seconde injection a été réalisée trois jours après. **B** : Dix-huit souris C57BL/6 ont été injectées avec 0.5M de cellules MC38 en sous-cutané. Trois jours plus tard, six souris ont été injectées avec ZZ-DTRBD_{HEK} en tampon PBS, six autres ont été injectées avec ZZ-DTRBD_{HEK} (2 nmole par souris) dans un tampon PBS contenant le mélange adjuvant CpG-B 1018/Poly I:C (30 µg pour chaque adjuvant) et six été non injectées (Contrôles). Deux injections additionnelles ont été réalisées à trois jours d'intervalle. La croissance tumorale a été suivie par mesure des tumeurs au pied à coulisse. Chaque injection est représentée par une flèche.
**Fig. 11**

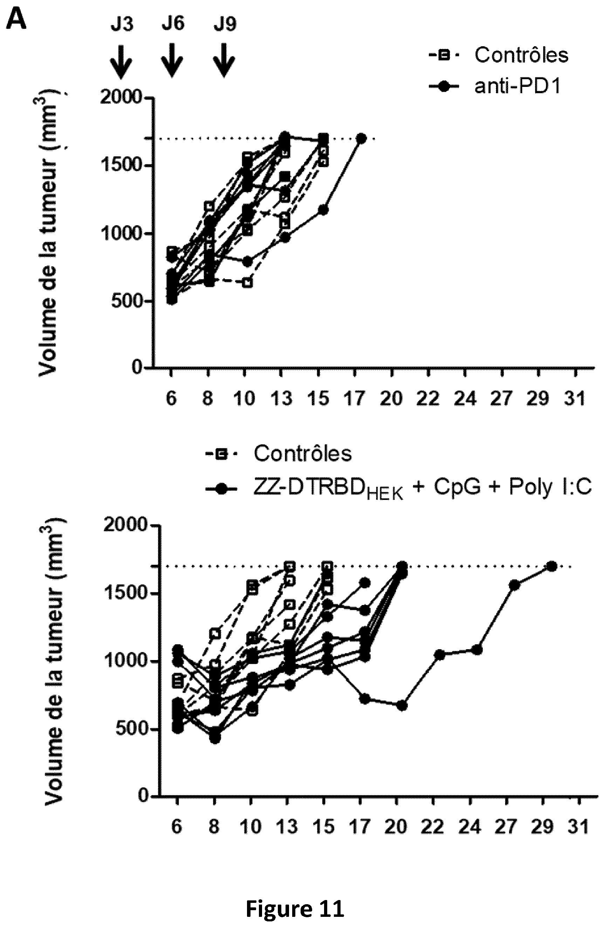

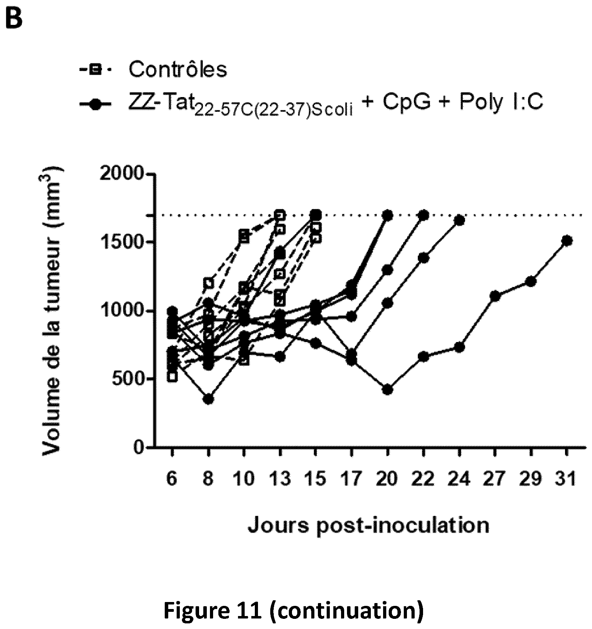

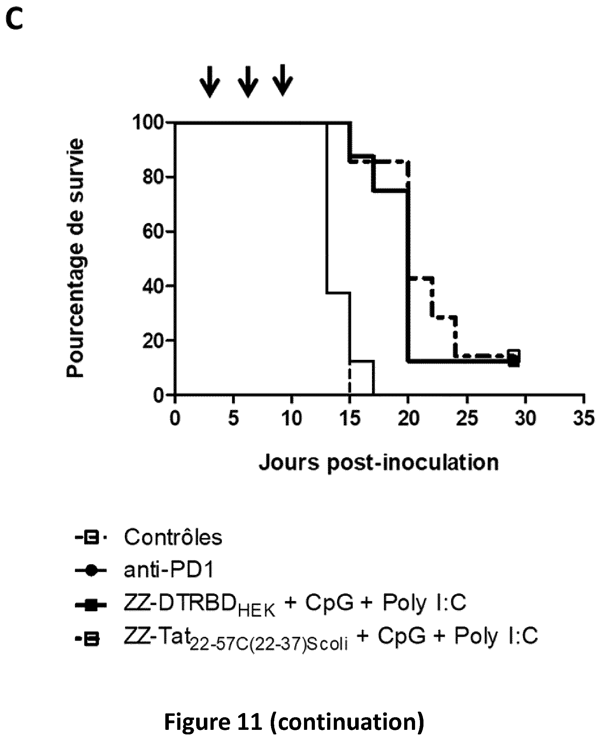

   [Fig. 11] montre que des souris injectées avec des cellules de cancer colorectal présentent une croissance tumorale ralentie lorsqu'elles sont ultérieurement injectées avec ZZ-DTRBD_{HEK}, ZZ-Tat_{22-57C(22-37)S} en association avec un mélange adjuvant. Trente-deux souris C57BL/6 ont été injectées avec 0.5M de cellules MC38 en sous-cutané. Trois jours plus tard, un groupe de huit souris a été injecté avec ZZ-DTRBD_{HEK} (0,96 nmole par souris) dans 50µl de PBS contenant le mélange adjuvant CpG-B 1018/Polyl:C (30 µg pour chaque adjuvant), un autre groupe avec un Ac anti-PD-1 (50µl, 1,3 nmole par souris), un troisième groupe avec ZZ-Tat_{22-57C(22-37)S} (0,96 nmole par souris) dans 50µl de PBS contenant le mélange adjuvant CpG-B 1018/Polyl:C (30 µg pour chaque adjuvant), et un dernier groupe de huit souris a été non injecté (Contrôles). Deux injections additionnelles ont été réalisées à trois jours d'intervalle. La croissance tumorale a été suivie par mesure des tumeurs au pied à coulisse. Chaque injection est représentée par une flèche.
   [Fig. 11A] Cinétique de croissance tumorale.
   [Fig. 11B] Cinétique de croissance tumorale.
   [Fig. 11C] Survie des animaux traités.
**Fig. 12**

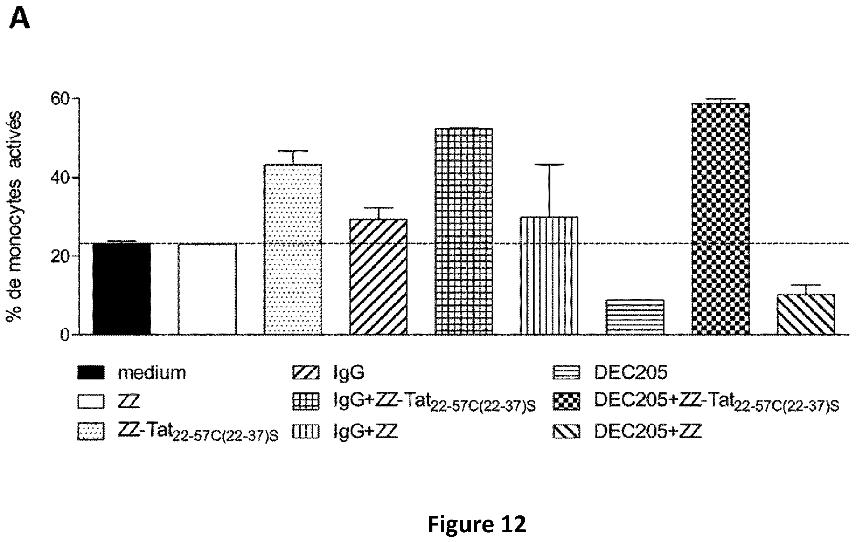

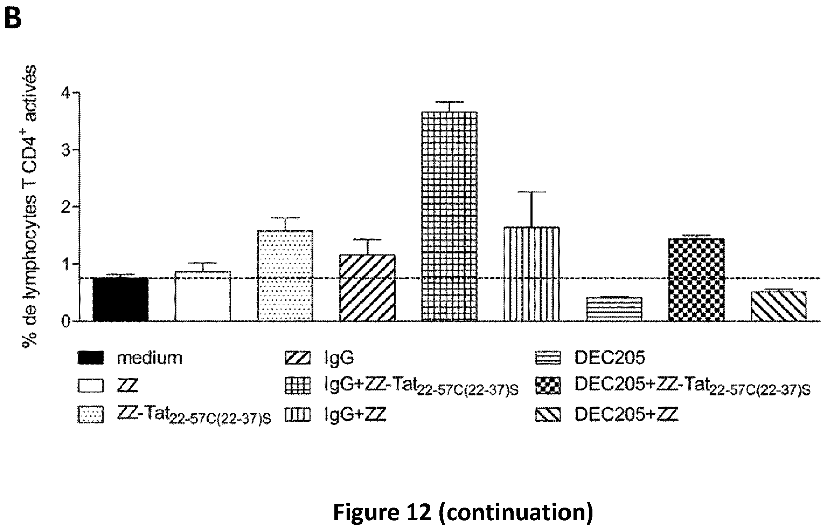

   [Fig. 12] montre que la formation des complexes moléculaires ZZ-Tat_{22-57C22-37)S}, anti-DEC205/ZZ-Tat_{22-57C22-37)S} et IgG/ZZ-Tat_{22-57C22-37)S} induisent *in vitro* l'augmentation de la proportion de monocytes et de lymphocytes T-CD4+ activés. Une concentration fixe (0,1µM) d'anti-DEC205/ZZ-Tat_{22-57C22-37)S}, IgG/ZZ-Tat_{22-57C22-37)S}, anti-DEC205/ZZ, IgG/ZZ, Ac anti-DEC205 a été incubée avec des CMSP humaines. Une concentration fixe (1µM) de ZZ-Tat_{22-57C22-37)S}, ou ZZ a été incubée avec des CMSP humaines. Après 24h les cellules ont été récoltées et marquées à l'aide d'Ac fluorescents. La proportion de monocytes (CD3⁻CD14⁺) activés dans la population de CMSP vivantes a été évaluée à l'aide du marqueur CD69.
   [Fig. 12A]. La proportion de lymphocytes T-CD4+ activés dans la population de lymphocytes T CD4+ (CD3⁺CD4⁺) vivants a été évaluée par mesure de l'expression de la molécule CD69.
   [Fig. 12B]. Les cellules ont été analysées par cytométrie de flux.
**Fig. 13**

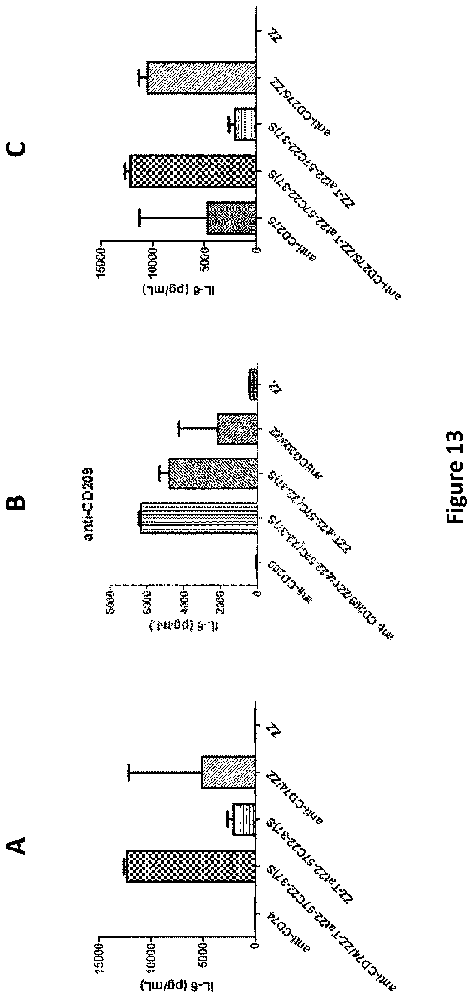

   [Fig. 13] montre que des complexes moléculaires dirigés contre des récepteurs exprimés par les cellules dendritiques (anti-CD74/ZZ-Tat_{22-57C22-37)S}, anti-CD209/ZZ-Tat_{22-57C22-37)S}, et anti-CD275/ZZ-Tat_{22-57C22-37)S}) induisent la sécrétion d'IL-6 par des CMSPs. Des CMSPs humaines ont été incubées en présence ou en absence des formes libres des Ac, de ZZ ou de ZZ-Tat_{22-57C(22-37)S} ainsi que des complexes moléculaires ciblant respectivement les molécules CD74 (A), CD209 (B) et CD275 (C). Après 24h les surnageants ont été prélevés et la présence d'IL-6 a été évaluée par dosage immunoenzymatique.
**Fig. 14**

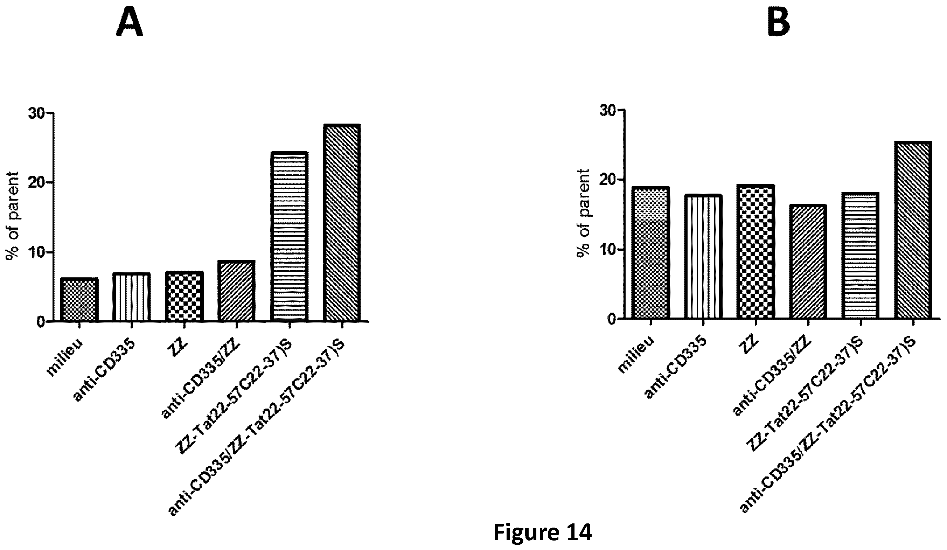

   [Fig. 14] montre qu'un complexe moléculaire (anti-CD335/ZZ-Tat_{22-57C22-37)S}) dirigé contre le récepteur CD335 exprimé par les cellules NK et NKT induit une augmentation de la proportion de cellules NK et NKT activées. Des CMSPs ont été incubées en présence ou en absence des formes libres des Ac, de ZZ ou de ZZ-Tat_{22-57C(22-37)S} ainsi que des complexes moléculaires anti-CD335/ZZ-Tat_{22-57C22-37)S}, anti-CD335/ZZ. Après 18h les cellules ont été analysées par cytométrie pour évaluer l'expression de la molécule de co-stimulation CD69 à la surface des cellules NK **(A)** et NKT **(B).**
**Fig. 15**

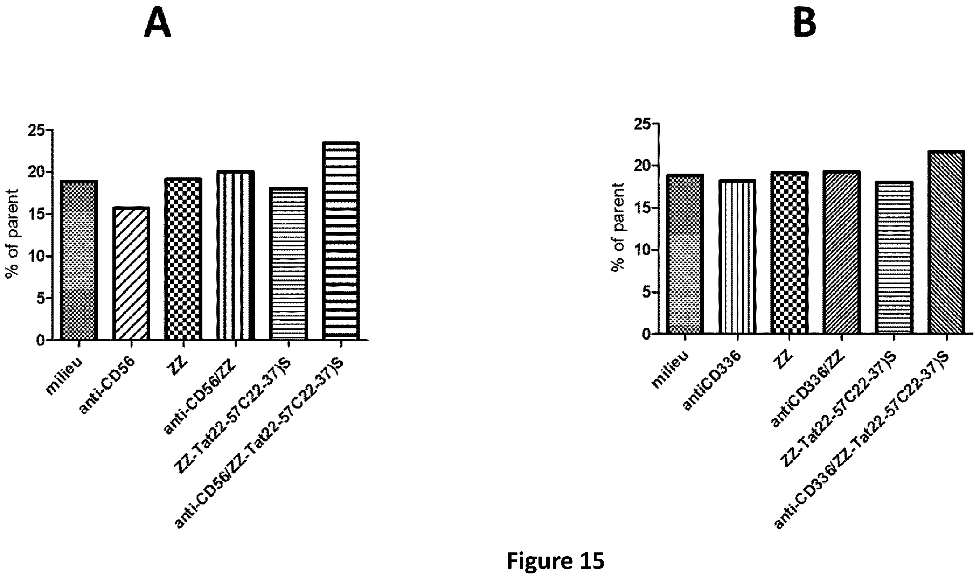

   [Fig. 15] montre que des complexes moléculaires respectivement dirigés contre deux récepteurs exprimés par les cellules NK et NKT induisent une augmentation de la proportion de cellules NKT activées. Des CMSPs ont été incubées en présence ou en absence des formes libres des Ac, de ZZ ou de ZZ-Tat_{22-57C(22-37)S} ainsi que des complexes moléculaires anti-CD56/ZZ-Tat_{22-57C22-37)S}, anti-CD56/ZZ, anti-CD336/ZZ-Tat_{22-57C22-37)S}, ou anti-CD336/ZZ. Après 18h les cellules ont été analysées par cytométrie pour évaluer l'expression de la molécule de co-stimulation CD69 à la surface des cellules NKT.
**Fig. 16**

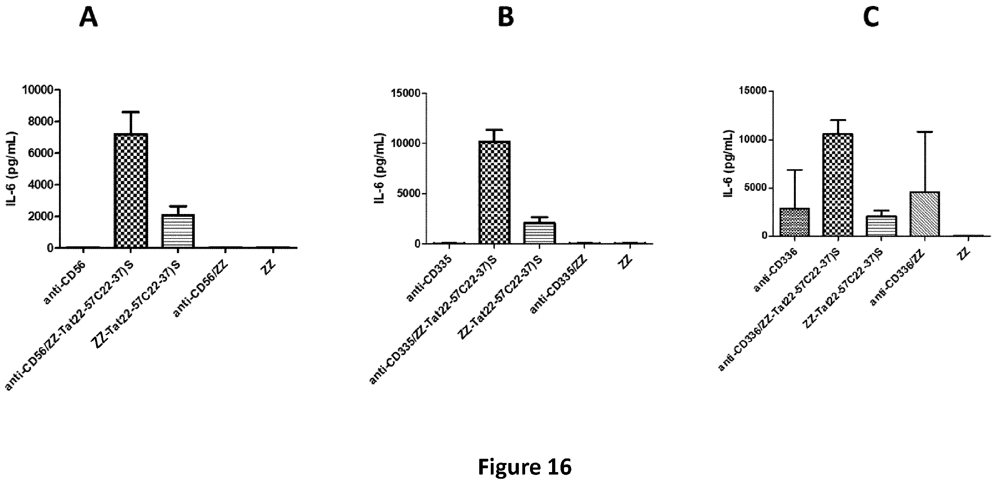

   [Fig. 16] montre que des complexes moléculaires dirigés contre des récepteurs exprimés par les cellules NK (anti-CD56/ZZ-Tat_{22-57C22-37)S}, anti-CD335/ZZ-Tat_{22-57C22-37)S}, et anti-CD336/ZZ-Tat_{22-57C22-37)S}) induisent la sécrétion d'IL-6 par des CMSPs humaines. Des CMSPs ont été incubées en présence ou en absence des formes libres des Ac, de ZZ ou de ZZ-Tat_{22-57C(22-37)S} ainsi que des complexes moléculaires ciblant respectivement les molécules CD56 **(A),** CD335 **(B)** et CD336 **(C).** Après 24h les surnageants ont été prélevés et la présence d'IL-6 a été évaluée par dosage immunoenzymatique.
**Fig. 17**
   [Fig. 18] montre que des complexes moléculaires contenant des Ac anti-ICPs (anti-CTLA-4/ZZ-Tat_{22-57C22-37)S,} anti-PD-L /ZZ-Tat_{22-57C22-37)S}, et anti-OX40/ZZ-Tat_{22-57C22-37)S}) induisent la sécrétion d'IL-6 par des CMSPs humaines. Des CMSPs ont été incubées en présence ou en absence des formes libres des Ac, de ZZ ou de ZZ-Tat_{22-57C(22-37)S} ainsi que des complexes moléculaires ciblant respectivement les molécules CTLA-4 **(A)**, PD-L1 **(B)** et OX40 **(C)**. Après 24h les surnageants ont été prélevés et la présence d'IL-6 a été évaluée par dosage immunoenzymatique

### Exemples

### Exemple 1 : Expression des protéines de fusion chez E. coli ou en cellules HEK, purification, caractérisation biochimique, et étude de leur capacité à lier les héparanes sulfates

### Matériels et Méthodes

### Production des différents complexes moléculaires

L'expression de la protéine de fusion ZZ-DTRBD chez *E. coli,* appelée ZZ-DTRBD_{coli}, a précédemment été décrite dans la publication de Lobeck et al. (Infection and Immunity, 1998, 66, 418-423). La protéine de fusion ZZ-DTRBD_{coli} (SEQ ID NO : 12) est codée par un polynucléotide de séquence SEQ ID NO : 11. L'expression de la protéine de fusion ZZ-Tat_{22-57C(22-37)S} chez *E. coli* a été réalisée en suivant un protocole similaire à celui décrit pour la fusion ZZOVATat_{22-57S} dans la publication de Knittel et al. (Vaccine, 2016, 34(27):3093-3101). La protéine de fusion ZZ-Tat_{22-57C(22-37)Scoli} (SEQ ID NO : 20) est codée par un polynucléotide de séquence SEQ ID NO : 19. Pour l'expression en cellules eucaryote des cellules HEK (2,5. 10⁶ cellules/ml dans 250ml milieu 293F freestyle) ont été transfectées avec un plasmide pCDNA3.4 codant pour ZZ-DTRBD_{HEK} (400µg de préparation d'ADN issu de maxiprep par transfection) en présence de PEI (0,5mg/ml). Ce plasmide comprend le polynucléotide de séquence SEQ ID NO : 13 qui code pour la protéine de fusion ZZ-DTRBD_{HEK} (SEQ ID NO : 14). Les cellules ont ensuite été incubées pendant 24h à 37°C sous agitation. 250ml de milieu Ex Cell a ensuite été ajouté. Après 4 jours d'incubation à 37°C sous agitation, les surnageants de culture ont été récupérés, filtrés stérilement et un cocktail d'inhibiteur de protéase a été ajouté.

Après expression des trois protéines, les surnageants ont respectivement été dilués au ½ en PBS-Tween 0,1% puis passés sur une colonne d'IgG sepharose (IgG sepharose 6Fast flow #17-0969-02, Amersham) afin de purifier les complexes moléculaires par immunoaffinité. L'acidité des protéines de fusion éluées de la colonne a été neutralisée en tampon Tris-HCl 1M, pH8. Les proteines de fusion issues de l'expression chez *E. coli,* ZZ-DTRBD_{coli} et ZZ-Tat_{22-57C(22-37)S}, ont subi un second cycle de purification en utilisant une colonne échangeuse de cations mono S 5/50 (GE Healthcare). La colonne a été équilibrée avec tampon phosphate citrate 0.05M pH=5.5 pour la purification de ZZ-DTRBD_{coli}. La colonne a été équilibrée avec tampon phosphate citrate 0.05M pH=4 pour la purification de ZZ-Tat_{22-57C(22-37)S}. Les protéines de fusion ZZ-DTRBD_{coli} et ZZ-Tat_{22-57C(22-37)S} ont ensuite été éluées avec un gradient linéaire de 0 à 1M NaCl._Les protéines ont finalement été concentrées en PBS et conservées à -20°C jusqu'à utilisation. La production des complexes moléculaires BB-DTRBD_{HEK} et BB-DTRBD_{coli} a été effectuée en suivant le même protocole que celui utilisé pour ZZ-DTRBD_{HEK} et ZZ-DTRBD_{coli}. La protéine de fusion BB-DTRBD_{HEK}(SEQ ID NO : 18) est codée par le polynucléotidede séquence SEQ ID NO : 17. La protéine de fusion BB-DTRBD_{coli} (SEQ ID NO : 16) est codée par le polynucléotidede séquence SEQ ID NO : 15.

### Analyse de la masse moléculaire et du degré d'homogénéité des complexes moléculaires ZZ-DTRBD_{HEK}, ZZ-DTRBD_{coli}, BB-DTRBD_{HEK} et BB-DTRBD_{coli} par gel d'électrophorèse

Les protéines ZZ-DTRBD_{HEK}, ZZ-DTRBD_{coli,}, BB-DTRBD_{HEK} et BB-DTRBD_{coli} ainsi que des marqueurs de poids moléculaires ont été déposés sur un gel SDS-PAGE 4-12% en conditions dénaturantes puis soumis à une migration électrophorétique. Suite à la migration, la présence de bandes protéiques a été révélée par coloration au bleu de coomasie.

### Liaison des complexes moléculaires à l'héparine

Pour évaluer la liaison des complexes moléculaires aux héparanes sulfates, l'héparine qui est un sucre sulfaté représentatif de la famille des héparane sulfates, a été utilisée.

L'interaction a été évaluée par une technique immunoenzymatique. Pour cela, une série de plaques de microtitration a été préalablement adsorbée avec des IgG de lapin (1µg/100µl/puits en tampon phosphate 0,1M pH7,2) puis saturées avec une solution tampon contenant de la sérum albumine bovine à 0,3 % (200µl/puits en tampon phosphate 0,1M pH7,2). Une autre série de plaques de microtitration a été saturée avec une solution tampon contenant de la sérum albumine bovine à 0,3 % (300µl/puits en tampon phosphate 0,1M pH7,2). Les deux séries de plaques ont ensuite été lavées et des séries de dilutions (en tampon phosphate 0,1M pH7,4 contenant 0,1% de sérum albumine bovine) des protéines ZZ-DTRBD_{HEK}, ZZ-DTRBD_{coli,}, BB-DTRBD_{HEK} et BB-DTRBD_{coli} et ZZ-Tat_{22-57C(22-37)S} et de ZZ libre ont été déposées dans les puits. Après 4 heures d'incubation à température ambiante les plaques ont été lavées et 100µl d'héparine-biotine (1µM) a été ajouté par puits. Après 1 heure à température ambiante les plaques ont été lavées et 100µl de streptavidine couplé à la péroxydase (dilution 1/2000) a été ajouté. Après 30 minutes d'incubation les plaques ont été lavées et un substrat (ABTS) a été ajouté. La coloration a été mesurée à 414 nm après 30 minutes d'incubation. Pour éliminer la liaison non-spécifique à l'albumine, le signal optique mesuré sur les plaques adsorbées uniquement avec la sérum albumine bovine a été déduit du signal mesuré sur les plaques adsorbées avec les IgG. La liaison à l'héparine est considérée significative quand le signal de densité optique s'avère supérieur ou égal à 50% du signal mesuré quand ZZ-Tat_{22-57C(22-37)S} est incubé à 100nM pH7,2 sur les plaques de microtitration.

### Résultats

Les inventeurs ont précédemment construit une protéine de fusion, appelée ZZ-DTRBD, incorporant, d'une part, un double domaine ZZ dérivé de la protéine A de *Staphylococcus aureus* et, d'autre part, le domaine DTRBD dérivé de la toxine diphtérique (Lobeck et al., Infection and Immunity, 1998, 66, 418-423). ZZ peut se lier à la région Fc des immunoglobulines de façon semblable à la protéine A. DTRBD lie quant à lui le récepteur de la toxine diphtérique et possède aussi un site de liaison aux héparanes sulfates localisé dans la région 453-467 (Knittel et al. J. Immunol., 2015, 194(8) :3601-11 ; Knittel et al. Vaccine, 2016, 34(27) :3093-3101). Ces caractéristiques de liaison permettent à ZZ-DTRBD un ciblage de différents types cellulaires porteurs d'immunoglobulines de surface et des héparanes sulfates protéoglycanes via l'interaction avec les héparanes sulfates.

Les inventeurs ont de la même façon construit une protéine de fusion, appelée BB-DTRBD, en remplaçant la séquence codant ZZ par une séquence codante dénommée BB. La protéine BB correspond à un double domaine qui est, tout comme ZZ, dérivé de la protéine A de *Staphylococcus aureus* mais présente la particularité de lier la région Fc ainsi que la région Fab des immunoglobulines (Jansson 1998 FEMS Immunol. and Med. Microbiol., Léonetti et al. 1999, J. Exp. Med., 189, 1217-28).

Les inventeurs ont aussi construit une protéine de fusion, appelée ZZ-Tat_{22-57C(22-37)S}. Elle contient un double domaine ZZ dérivé de la protéine A de *Staphylococcus aureus* et un domaine Tat_{22-57C(22-37)S} dérivé du transactivateur transcriptionnel du VIH (WO 2011/092675, et Knittel et al. Vaccine, 2016, 34(27) :3093-3101) qui possède un site de liaison aux héparanes sulfates. Ces caractéristiques de liaison permettent à ZZ-Tat_{22-57C(22-37)S} un ciblage de différents types cellulaires porteurs d'immunoglobulines de surface et des héparanes sulfates protéoglycanes via l'interaction avec les HS.

Les inventeurs ont exprimé ZZ-DTRBD, BB-DTRBD et ZZ-Tat_{22-57C(22-37)S} par voie recombinante à l'aide de différents systèmes d'expression. Les complexes exprimés chez *E. coli* sont appelés ZZ-DTRBD_{coli}, BB-DTRBD_{coli} et ZZ-Tat_{22-57C(22-37)S} et ceux exprimés en cellules HEK sont dénommés ZZ-DTRBD_{HEK} et BB-DTRBD_{HEK}. Après expression, les complexes ont été purifiés à l'aide d'une colonne contenant un gel porteur d'IgG. Ensuite les protéines ZZ-DTRBD_{coli}, BB-DTRBD_{coli} et ZZ-Tat_{22-57C(22-37)S} ont été soumises à une chromatographie par échange d'ions afin d'éliminer le LPS contaminant. Enfin, certaines caractéristiques de ces complexes ont été évaluées par gel d'électrophorèse. Comme on peut le voir sur la **Fig. 1****,** ZZ-DTRBD_{coli} et BB-DTRBD_{coli} migrent en une bande prédominante avec une masse moléculaire d'environ 35kDa qui est proche de la masse théorique de la molécule (32230) ce qui indique que cette bande correspond à un monomère. Des bandes minoritaires correspondant à des produits de dégradation sont aussi présentes chez ces deux complexes moléculaires. ZZ-DTRBD_{HEK} et BB-DTRBD_{HEK} migrent pour leur part en plusieurs bandes essentiellement distribuées entre 35 et 150k Da ce qui indique que l'expression en cellules HEK conduit à un mélange hétérogène constitué de formes monomériques et oligomériques de ZZ-DTRBD. Le complexe moléculaire ZZ-Tat_{22-57C(22-37)S} migre pour sa part en une bande majoritaire qui reflète l'homogénéité de la protéine purifiée. La masse moléculaire d'environ 25kDa est un peu supérieure à celle calculée pour la masse théorique de la molécule (MW=19145). Cependant, Tat et ses dérivés ont tendance à migrer de façon anormale (Kittiworakarn et al. etc) ce qui suggère fortement que la protéine de fusion ZZ-Tat_{22-57C(22-37)S} exprimée chez *E. coli* et purifiée est essentiellement constituée par un monomère.

Ensuite, les inventeurs ont étudié l'aptitude de ZZ libre et des cinq complexes moléculaires à lier l'héparine, qui est un polysaccharide sulfaté représentatif de la famille des héparanes sulfates. Le dosage immunoenzymatique ne permet pas d'observer un signal optique significatif pour ZZ libre (Fig. 2). En revanche, un accroissement de la densité optique en fonction de la dose incubée est mesuré pour ZZ-DTRBD_{coli}, BB-DTRBD_{HEK}, BB-DTRBD_{coli}, ZZ-DTRBD_{HEK} etZZ-Tat_{22-57C(22-37)S.} Ces données indiquent donc que la présence du domaine DTRBD ou du domaine Tat_{22-57C(22-37)S} permet la liaison des complexes moléculaires à l'héparine. De plus, comme les fusions ZZ-DTRBD_{HEK} et BB-DTRBD_{HEK} contiennent des oligomères (cf **figure 1****)** ces données indiquent que la présence d'oligomères ne perturbe pas l'interaction.

### Exemple 2 : La protéine de fusion ZZ-DTRBD_{HEK} est capable de lier différents types de cellules du système immunitaire murin

### Matériels et méthodes

### Liaison aux cellules du système immunitaire de ZZ-DTRBD

Des splénocytes de souris C57BI/6 sont resuspendus à 10.10⁶ cellules/mL en tampon PBS 0,5% BSA 2mM EDTA. 100 µL de la suspension cellulaire sont disposés en plaque 96 puits fond rond. 100 µL de tampon 0,5% BSA 2 mM EDTA sont ajoutés par puits en absence ou en présence de ZZ-DTRBD_{HEK} (100 nM). Les mélanges sont incubées 30 minutes à 4°C puis lavées 2 fois en PBS 0,5% BSA 2 mM EDTA. 2 µg par puits d'IgG de lapin sont ajoutés sur les cellules qui sont incubées 20 minutes à 4°C puis lavées 2 fois qsp 200 µL en PBS 0,5% BSA 2 mM EDTA. Les cellules sont resuspendues dans 50 µL de tampon de marquage (PBS 0,5% BSA 2 mM EDTA) contenant différents mélanges d'Ac de chez BioLegend. Les mélanges sont incubés 20 minutes à 4°C à l'abri de la lumière puis les splénocytes sont lavés 2 fois qsp 200 µL en PBS 0,5% BSA 2 mM EDTA. Les cellules sont ensuite fixées pendant 30 minutes à température ambiante. Pour cela, 100 µL de tampon 4% PFA puis 100 µL de PBS 0,5% BSA 2 mM EDTA ont été ajoutés avant acquisition sur cytomètre BD FACSAria^{™}.

Mélange 1 pour les cellules dendritiques (duplicats) : B220-FITC (#103206, 1/200), CD11c-PE-Cy7 (#117318, 1/200), CD317-APC (#127016, 1/100), CD8a-PerCP-Cy5.5 (#100734, 1/200), CD11b-APC-Cy7 (#101226, 1/800), Donkey anti-rabbit-BV421 (#406410, 1/100), Live Dead Aqua (ThermoFischer #L34966, 1/1000).

Mélange 2 pour les monocytes et lymphocytes T, B (duplicats) : B220-FITC (#103206, 1/200), CD19-BV650 (#115541, 1/100), CD3-APC-Cy7 (#100222, 1/100), CD4-BV605 (#100451, 1/200), NK1.1-PE-Cy7 (#108714, 1/100), CD8a-PerCP-Cy5.5 (#100734, 1/200), CD11b-APC (#101212, 1/800), Ly6C-PE #128007, 1/800), Donkey anti-rabbit-BV421 (#406410, 1/100), Live Dead Aqua (ThermoFischer #L34966, 1/1000).

### Résultats

Pour évaluer la capacité d'un complexe moléculaire à lier des cellules du système immunitaire, ZZ-DTRBD_{HEK} a été incubé en présence de splénocytes de souris. Il a ainsi pu être observé que cette protéine de fusion se lie préférentiellement aux cellules cDC-CD8+ qui sont des CPAg spécialisées (Fig. **3A****).** Elle interagit aussi préférentiellement avec les monocytes qui peuvent aussi jouer le rôle de CPAg **(****Fig. 3B****).** Enfin, elle se lie dans de moindres proportions aux lymphocytes. Ces données indiquent donc que la protéine de fusion cible préférentiellement les CPAg.

### Exemple 3 : Les protéines de fusion ZZ-DTRBD_{HEK} et ZZ-Tat_{22-57C(22-37)S} induisent in vitro la sécrétion d'IL-6 et d'IL-12 par des cellules du système immunitaire.

### Matériels et Méthodes

Des splénocytes de souris C57BL/6 sont resuspendus à raison de 2.10⁶ cellules/mL en milieu RPMI 10% SVF 1% Pénicilline/Streptomycine. 100 µL de la suspension cellulaire sont répartis en plaque 96 puits en présence ou en absence des complexes moléculaires (ZZ, DTRBD, ZZ-DTRBD_{HEK}, ou ZZ-Tat_{22-57C(22-37)S)} incubés à 1 µM final. Après 24h d'incubation, les surnageants sont récoltés pour un dosage des cytokines IL-6 et IL-12 par ELISA réalisé selon les instructions du fabricant (R&D #DY406-05 et #DY419).

### Résultats

Les inventeurs se sont demandés si les complexes moléculaires peuvent induire l'activation de cellules du système immunitaire. Comme l'activation cellulaire peut conduire à la sécrétion de cytokines, les inventeurs ont décidé d'évaluer *in vitro* la présence de deux d'entre elles dans des surnageants issus de l'incubation de splénocytes de souris avec des complexes moléculaires et différentes protéines contrôle. La première cytokine est l'IL-6 car elle est représentative des cytokines inflammatoires importantes pour l'initiation de la réponse immunitaire. La seconde est l'IL-12 car elle s'avère cruciale pour l'induction des réponses immunitaires cellulaires. Ils ont trouvé ces deux cytokines en quantité accrue dans les surnageants issus de l'incubation avec ZZ-DTRBD_{HEK} ce qui indique que ce complexe moléculaire induit l'activation des splénocytes **(****Fig. 4****).** En revanche, ils n'ont pas retrouvé d'accroissement de la quantité de cytokines dans les surnageants issus de l'incubation avec ZZ libre ou DTRBD libre ce qui indique que le double domaine de liaison aux Ig et le domaine de liaison aux héparanes sulfates ne peuvent activer, à eux seul, des cellules immunitaires. Ces résultats démontrent donc que ZZ-DTRBD_{HEK} est capable d'induire l'activation de cellules du système immunitaire et indiquent que l'association du double domaine de liaison aux Ig et du domaine DTRBD de liaison aux HS est absolument requise pour l'effet.

Les inventeurs ont procédé selon un principe similaire à celui décrit dans le paragraphe précédent pour évaluer si ZZ-Tat_{22-57C(22-37)S} est capable d'activer des cellules du système immunitaire et si l'association de ZZ et du domaine de Tat impliqué dans la liaison aux héparanes sulfates est aussi absolument requise pour l'effet stimulateur. Pour cela, ils ont notamment utilisé un peptide, nommé Tat_{CY49-57}, qui contient la région basique de Tat et s'avère de ce fait représentatif de l'interaction de Tat et de ses dérivés avec les héparanes sulfates. Ils ont incubé des splénocytes de souris avec ZZ, Tat_{CY49-57}, ZZ + Tat_{CY49-57},et ZZ-Tat_{22-57C(22-37)S}, respectivement. Ils ont ensuite évalué la présence d'IL-6 et IL-12 dans les surnageants. Ils ont trouvé ces deux cytokines dans les surnageants issus de l'incubation avec ZZ-Tat_{22-57C(22-37)S} mais pas dans les surnageants issus de l'incubation avec ZZ, Tat_{CY49-57}, ZZ + Tat_{CY49-57} **(****Fig** . **5****).** Ces résultats démontrent donc que ZZ-Tat_{22-57C(22-37)S} est capable d'induire l'activation de cellules du système immunitaire et que cette caractéristique n'est pas partagée par les formes libres ZZ et Tat_{CY49-57} ce qui indique que l'association du double domaine de liaison aux Ig et de la région basique de Tat responsable dans la liaison aux HS est absolument requise pour l'effet.

### Exemple 4 : Un complexe moléculaire i) reste capable de déclencher la sécrétion d'IL-6 et d'IL-12 par des cellules du système immunitaire lorsque son domaine ZZ est substitué par BB, ii) présente des capacités stimulantes accrues lorsqu'il est constitué de formes oligomériques.

### Matériels et Méthodes

Des splénocytes de souris C57BL/6 sont resuspendus à raison de 2.10⁶ cellules/mL en milieu RPMI 10% SVF 1% Pénicilline/Streptomycine. 100 µL de la suspension cellulaire sont répartis en plaque 96 puits en présence ou en absence de différents complexes moléculaires (ZZ-DTRBD_{HEK}, BB-DTRBD_{HEK}, ZZ-DTRBD_{coli} et BB-DTRBD_{coli}) incubés à 1 µM final. Après 24h d'incubation, les surnageants sont récoltés pour un dosage des cytokines IL-6 et IL-12 par ELISA réalisé selon les instructions du fabricant (R&D #DY406-05 et #DY419).

### Résultats

Les travaux décrits dans l'exemple 3 ont été réalisés avec deux complexes moléculaires contenant le double domaine ZZ. Ce double domaine qui lie le Fc des Ac peut ainsi cibler les Ac localisées à la surface des CPAg. Les inventeurs se sont ensuite demandés si des complexes moléculaires pouvant cibler conjointement la région Fc et la région Fab des Ig peuvent aussi être capables d'activer des cellules du système immunitaire. Pour cela, ils se sont fondés sur le double domaine BB dérivé de la protéine A de *Staphylococcus aureus,* qui présente la particularité de pouvoir lier la région Fc des Ac de la même manière que Z mais aussi la région Fab des Ac, contrairement à ZZ (Jansson B. et al., Fems Immunol. Med. Microbiol. 1998, 20 :69-78). Ils ont préparé les complexes BB-DTRBD_{HEK} et BB-DTRBD_{coli} qui sont décrits dans l'exemple 1 et les ont comparé à ZZ-DTRBD_{HEK} et ZZ-DTRBD_{coli} pour la capacité à induire la sécrétion de cytokines IL-6 et IL-12 *in vitro* lorsqu'ils sont incubés avec des splénocytes murins.

Comme les inventeurs se sont aperçus que ZZ-DTRBD et BB-DTRBD sont sous forme oligomérique suite à leur expression en cellules HEK (cf. **Fig. 1****)** alors qu'ils sont monomérique après expression chez *E. coli* ces travaux permettaient aussi d'évaluer si l'état d'oligomérisation peut affecter la capacité à induire le système immunitaire.

Les inventeurs se sont rendus compte que les surnageants issus de l'incubation avec ZZ-DTRBD_{HEK} contiennent de plus fortes quantités de cytokines que ceux issus de l'incubation avec BB-DTRBD_{HEK} **(****fig. 6****).** De la même manière, les surnageants issus de l'incubation avec ZZ-DTRBD_{coli} contiennent de plus fortes quantités de cytokines que ceux issus de l'incubation avec BB-DTRBD_{coli}. Ces résultats démontrent qu'un complexe moléculaire contenant BB reste capable d'induire la sécrétion de cytokines *in vitro,* mais dans des proportions moindre qu'un complexe moléculaire contenant ZZ. Ces données indiquent donc que l'effet d'activation peut être médié par des complexes moléculaires contenant des domaines capables de lier différents sites sur les Ac.

La comparaison des taux de cytokines dans les surnageants en fonction du type cellulaire utilisé (i.e. HEK versus *E. coli*) pour la production des complexes moléculaires a permis de mettre en évidence la différence d'efficacité stimulante en fonction du type de production. En effet, dans les surnageants issus de l'incubation avec les complexes provenant de cellules HEK (ZZ-DTRBD_{HEK}, BB-DTRBD_{HEK}) l'IL-6 et l'IL-12 sont présentes en quantité plus importante que dans ceux issus de l'incubation avec les complexes issus de l'expression chez *E. coli* (ZZ-DTRBD_{coli} et BB-DTRBD_{coli}) (**Fig. 6****)**. Comme les protéines issues de l'expression en cellules HEK sont principalement oligomériques (cf. **Fig. 1****),** les inventeurs en ont déduit que le système immunitaire est plus efficacement induit quand les complexes moléculaires présentent un taux d'oligomérisation élevé.

### Exemple 5 : Des cellules dendritiques isolées sécrètent de l'IL-6 et de l'IL-12 lorsqu'elles sont incubées en présence de ZZ-DTRBD_{HEK}

### Matériels et méthodes

Une souris C57BL/6J est euthanasiée puis sa rate est récupérée en milieu RPMI 10% SVF 1% Pénicilline/Streptomycine. La rate est perfusée avec 3 mL de collagénase D à 2 mg/mL en HBSS 0,5% BSA puis incubée 30 minutes à 37°C. Les splénocytes sont récupérés et un tri magnétique des cellules dendritiques (DC) est effectué selon les instructions du fabricant (Miltenyi Biotec #130-100-875). Les DC sont centrifugées 5 minutes à 4°C à 390g puis resuspendues à 2.10⁶ cellules/mL en milieu RPMI 10% SVF 1% Pénicilline/Streptomycine. 100 µL de la suspension cellulaire (200 000 cellules) sont disposés en plaque 96 puits fond plat et 100 µL de milieu RPMI 10% SVF 1% Pénicilline/Streptomycine est ajouté en absence ou présence de ZZ-DTRBD_{HEK} (0,6 µM final). Les cellules sont incubées 24h à 37°C, puis les surnageants sont récoltés pour un dosage des cytokines IL-6 et IL-12 par ELISA selon les instructions du fabricant (R&D #DY406-05 et #DY419).

### Résultats

La mise en place de mécanismes de défense immunitaire dépend de la collaboration entre différents partenaires cellulaires. Parmi ceux-ci, les cellules dendritiques (DC) représentent des CPAg jouent un rôle central. En effet, elles contribuent à l'activation d'autres types cellulaires via des interactions directes ou par l'intermédiaire de cytokines qu'elles sécrètent. Pour évaluer si ZZ-DTRBD induit la sécrétion de cytokines par des DC, des DC ont été purifiés à partir de splénocytes de souris C57BI/6. Ensuite, ces DC ont été incubées pendant 24h en présence ou en l'absence de ZZ-DTRBD puis les surnageants ont été prélevés afin de doser la présence d'IL-6 et d'IL-12 **(****Fig. 7****).** La quantité d'IL-6 présente dans les surnageants issus de l'incubation avec ZZ-DTRBD_{HEK} est environ 5 fois supérieure à celle trouvée dans les surnageants issus de l'incubation sans complexe moléculaire. Une quantité importante d'IL-12 (environ 8700pg/ml) est aussi détectée dans les surnageants issus de l'incubation avec ZZ-DTRBD_{HEK} mais quasiment pas dans ceux issus de l'incubation sans complexe moléculaire. Ces données indiquent donc que ZZ-DTRBD provoque la sécrétion d'IL-6 et d'IL-12 par des cellules dendritiques murines et pourrait ainsi contribuer à la mise en place de mécanismes de défense immunitaire.

### Exemple 6 : Les protéines ZZ-DTRBD, et ZZ-Tat_{22-57C(22-37)S} sont capables d'induire des cellules dendritiques chez la souris.

### Matériels et Méthodes

Trois groupes de huit souris C57BL/6 ont été injectés trois fois à trois jours d'intervalle avec 100 µl de PBS en absence ou en présence de ZZ-DTRBD_{HEK} (5 nmoles par souris) ou ZZ-Tat_{22-57C(22-37)S} (10 nmoles par souris), respectivement. 24 heures après la dernière injection les animaux ont été euthanasiés, les rates ont été collectées pour récupérer les splénocytes. Les cellules ont été resuspendues dans 50 µL de tampon de marquage (PBS 0,5% BSA 2 mM EDTA) contenant différents mélanges d'Ac. Les cellules ont ensuite été incubées 20 minutes à 4°C à l'abri de la lumière puis lavées 2 fois en PBS 0,5% BSA 2 mM EDTA. Les cellules ont ensuite été fixées pendant 20 minutes à 4°C dans 100 µL de tampon 4% PFA puis lavées en PBS 0,5% BSA 2 mM EDTA. Celles-ci ont finalement été resuspendues dans 200 µL de PBS 0,5% BSA 2 mM EDTA puis analysées à l'aide d'un cytomètre de flux BD FACSAria^{™}. Les cellules ont été analysées par cytométrie de flux. Les cDC-CD8+ sont identifiées CD11c^{high}B220⁻CD8⁺CD11b⁻, les cDC-CD11b+ sont identifiées CD11c^{high}B220⁻CD8⁻CD11b⁺ et les pDC sont identifiées CD11c^{int} CD317⁺CD11b⁺.

Les mélanges d'Ac utilisés pour l'analyse phénotypique des cellules dendritiques sont les suivants : B220-FITC (#103206, 1/200), CD11c-PE-Cy7 (#117318, 1/200), CD317-APC (#127016, 1/100), CD11b-APC-Cy7 (#101226, 1/800), CD8a-PerCP-Cy5.5 (#100734, 1/200), Live Dead Violet (ThermoFischer #L34964, 1/1000).

### Résultats

Comme les complexes moléculaires se lient préférentiellement aux CPAg *in vitro* et induisent la sécrétion de cytokines, les inventeurs se sont demandés si de tels complexes peuvent induire l'expansion des CPAg chez la souris. Pour évaluer cet aspect les inventeurs ont injecté trois groupes de 8 souris C57BI/6 avec une solution de PBS en présence ou en absence de ZZ-DTRBD_{HEK} ou ZZ-Tat_{22-57C(22-37)S}. Ils ont ensuite euthanasié les animaux et prélevé leur rate pour évaluer la fréquence des différentes sous-populations de cellules dendritiques dans les splénocytes. Comme on peut le voir sur la figure 8, les trois populations de DC murines (i.e. DC-CD8+, DC-CD11b+ et pDC) sont présentes dans les rates du groupe de souris injectées uniquement avec du PBS. La fréquence de ces trois types cellulaires est cependant significativement accrue dans les groupes d'animaux injectés avec ZZ-DTRBD_{HEK} ou ZZ-Tat_{22-57C(22-37)S}. Ces données indiquent donc que les complexes moléculaires sont capables d'induire l'expansion des cellules dendritiques *in vivo.* Comme ces cellules sont centrales dans l'initiation de la réponse immunitaire, ces résultats suggèrent fortement que les complexes peuvent ainsi favoriser l'induction de mécanismes de réponse immunitaire.

### Exemple 7 : ZZ-DTRBD_{HEK} provoque la sécrétion d'IL-6 et d'IL-12 par des cellules dendritiques humaines in vitro

### Matériel et Méthodes

### Isolement de cellules dendritiques humaines et incubation pour l'étude de la sécrétion d'IL-6 et d'IL-12.

Une poche de couche leuco-plaquétaire est diluée au ½ dans du milieu AIMV puis incubée pendant une nuit à température ambiante sous agitation. 15 mL de milieu histopaque est ensuite ajouté dans 4 tubes leucosep sur lesquels sont ajoutés 4 x 25 mL du sang dilué. Les tubes sont centifugés 15 minutes à température ambiante à 1000 g sans frein. Les anneaux de cellules mononuclées du sang périphérique (CMSP) sont récupérés et lavés au PBS 2 mM EDTA sans calcium ni magnésium. Les CMSP sont centrifugés à 150 g pendant 10 minutes à température ambiante puis un tampon de lyse des hématies (8,3 mg/mL NH₄Cl, 0,84 mg/mL NaHCOs, 0,1 mM EDTA) est ajouté et incubé 10 minutes à 4°C. 40 mL de PBS sont rajoutés puis les cellules sont centrifugées 10 minutes à température ambiante à 150 g. Le tri des DC est ensuite effectué selon les instructions du fabricant (Miltenyi Biotec #130-091-379). Les DC sont centrifugées 5 minutes à 4°C à 390g puis resuspendues à 2.10⁶ cellules/mL en milieu RPMI 10% SVF 1% Pénicilline/Streptomycine. 100 µl des DC triées (200 000 cellules) sont disposés en plaque 96 puits et 100 µL de milieu RPMI 10% SVF 1% Pénicilline/Streptomycine en absence ou présence de ZZ-DTRBD_{HEK} (1 µM final). Les cellules sont incubées 24h à 37°C puis les surnageants sont récoltés pour un dosage des cytokines IL-6 et IL-12 par ELISA selon les instructions du fabricant (R&D #DY206-05 et #DY1270-05).

### Résultats

Pour évaluer si ZZ-DTRBD_{HEK} peut induire *in vitro* la sécrétion d'IL-6 et d'IL-12 par des DC issues de donneurs humains sains, après 24h d'incubation la présence de ces deux cytokines a été évaluée dans les surnageants de culture de ces cellules. Comme on peut le voir sur la **Fig. 9****,** de l'IL-6 est trouvé en quantité environ 10 fois plus importante dans les surnageants issus de l'incubation en présence ZZ-DTRBD_{HEK} par rapport aux surnageants issus de l'incubation en absence de la protéine de fusion. L'IL-12 est quant à elle présente en grande quantité (2400pg/ml) dans les surnageants issus de l'incubation des DCs en présence ZZ-DTRBD_{HEK} et en très faible quantité (8 pg/ml) dans ceux issus de l'incubation en absence du complexe moléculaire. L'ensemble de ces données indique donc que ZZ-DTRBD induit une augmentation de la sécrétion d'IL-6 et d'IL12 par des DCs humaines. ZZ-DTRBD pourrait ainsi contribuer à la mise en place de mécanismes de défense immunitaire chez l'homme qui seraient liés à la sécrétion de ces cytokines.

### Exemple 8 : ZZ-DTRBD et ZZ-Tat_{22-57C(22-37)S} ralentissent la progression de la croissance d'une tumeur colorectale.

### Matériels et Méthodes

### Etude de l'effet du mélange adjuvant CpG/Polyl:C sur la croissance tumorale d'une lignée murine de cancer colorectal

Deux groupes de huit souris C57BL/6 ont été injectés avec 0.5M de cellules MC38 en sous-cutané dans la patte. Les souris ont ensuite été non injectées (Contrôles) ou injectées avec le mélange adjuvant CpG-B 1018/Poly I:C. (30 µg pour chaque adjuvant) trois et six jours plus tard. La croissance tumorale a été suivie par mesure des tumeurs au pied à coulisse. A l'atteinte du critère d'arrêt, elles ont été euthanasiées.

### Etude de l'effet du complexe moléculaire ZZ-DTRBD_{HEK}, injecté en absence ou en présence d'adjuvant, sur la croissance tumorale d'une lignée murine de cancer colorectal

Trois groupes de six souris C57BL/6 ont été injectés avec 0.5M de cellules MC38 en sous-cutané dans la patte. Trois, six et neuf jours après, les souris sont soit non injectées (Contrôles), soit injectées avec ZZ-DTRBD_{HEK} (2 nmoles par souris) en absence ou en présence de CpG-B 1018 + Poly I:C. (30 µg pour chaque adjuvant par souris). Les souris sont suivies individuellement pendant 15 jours (mesures de tumeurs avec un pied à coulisse) avant euthanasie.

### Comparaison de l'effet d'un Ac anti-PD-1 et des complexes moléculaires ZZ-DTRBD_{HEK} et ZZ-Tat_{22-57C(22-37)S} sur la croissance tumorale d'une lignée murine de cancer colorectal

Quatre groupes de huit souris C57BL/6 ont été injectés avec 0.5M de cellules MC38 en sous-cutané dans la patte. Trois, six et neuf jours après, les souris sont soit non injectées (Contrôles), soit injectées avec l'anticorps anti-PD-1 (Euromedex #BE0146-100MG, clone RMP1-14), ZZ-DTRBD_{HEK} ou ZZ-Tat_{22-57C(22-37)S} (0,96 nmole par souris) en présence de CpG-B 1018 + Poly I:C. (30 µg pour chaque adjuvant par souris). Les souris sont suivies individuellement (mesures de tumeurs avec un pied à coulisse) au cours du temps. A l'atteinte du critère d'arrêt, elles sont euthanasiées

### Résultats

Pour évaluer si les complexes moléculaires peuvent avoir un impact sur la croissance d'une tumeur des études ont été réalisées dans un modèle syngénique de cancer murin. Ce modèle est basé sur une lignée tumorale de colon, appelée MC38, et des souris C57BI/6. L'induction du cancer est provoquée par l'injection de 500000 cellules MC38 par souris.

Dans un premier temps, l'impact du mélange adjuvant CpG1018/polyl:C sur la croissance tumorale a été évalué. Comme on peut le voir sur la **Fig. 10A****,** par rapport au groupe non-traité (contrôles) la croissance tumorale n'est quasiment pas impactée chez le groupe de souris injecté avec le mélange CpG1018/polyl:C. Ces données indiquent donc que ce mélange adjuvant ne peut ralentir à lui seul la croissance de cette tumeur colorectale.

Dans un second temps, l'impact de ZZ-DTRBD_{HEK} injecté seul ou en présence du mélange adjuvant CpG1018/polyl:C sur la croissance tumorale a été évalué. Comme on peut le voir sur la **Fig. 10B****,** par rapport au groupe non-traité (contrôles) la croissance tumorale est ralentie au jour 8 et au jour 10 dans le groupe injecté avec ZZ-DTRBD_{HEK} sans adjuvant. L'effet s'avère encore plus important dans le groupe traité avec ZZ-DTRBD_{HEK} et le mélange adjuvant CpG1018/polyl:C. En effet, dans ce groupe la croissance tumorale est ralentie sur l'ensemble de la période d'observation des animaux (J6 à J15). L'ensemble de ces données indique donc que ZZ-DTRBD_{HEK} a un impact sur la croissance de la lignée MC38 et que cet effet est amplifié lorsqu'il est mélangé à l'adjuvant CpG1018/polyl:C.

Dans une autre série d'expérience, l'effet sur la croissance de la tumeur MC38 chez la souris C57BI/6 a été comparé pour les traitements suivants: ZZ-DTRBD_{HEK}/CpG1018/polyl:C, ZZ-Tat_{22-57C(22-37)S}/CpG1018/polyl:C, Ac anti-PD-1. Comme on peut le voir sur la **Fig. 11A** **et** **11B****,** le traitement avec l'Ac anti-PD-1 n'impacte pas significativement la croissance tumorale par rapport au groupe contrôle. Les souris respectivement injectées avec ZZ-DTRBD_{HEK}/CpG1018/polyl:C et ZZ-Tat_{22-57C(22-37)S}/CpG1018/polyl:C présentent en revanche une diminution de la croissance tumorale. Ce ralentissement de la croissance tumorale conduit à une survie accrue des animaux dans ces deux groupes **(****Fig. 11C****).**

### Exemple 9 : Des monocytes et des lymphocytes CD4+ activés peuvent être induits par des complexes moléculaires ciblant respectivement la molécule de surface DEC205 et des récepteurs Fc_{gamma}.

### Matériels et Méthodes

Pour préparer un complexe moléculaire ciblant la molécule DEC205 un Ac anti-DEC205 a été utilisé (BioLegend ; clone NLDC-145, ref BLE138202). Il a été incubé en absence ou en présence de ZZ-Tat_{22-57C22-37)S} à une concentration fixe (0,2µM pour chaque molécule), pendant 24 heures en milieu RPMI sans SVF. La capacité de la région ZZ à lier la région Fc de l'Ac anti-DEC205 a permis de former un complexe moléculaire non-covalent, appelé anti-DEC205/ZZ-Tat_{22-57C22-37)S}. Le même protocole a été utilisé pour former un complexe entre la molécule ZZ et l'Ac anti-DEC205, appelé anti-DEC205/ZZ.

Pour préparer un complexe moléculaire ciblant les récepteurs FC_{gamma} un Ac polyclonal humain non-spécifique a été utilisé. Cet Ac a été incubé avec ZZ-Tat_{22-57C22-37)S} ou ZZ en suivant un protocole identique à celui utilisé pour l'Ac anti-DEC205. Il a ainsi été possible de former un complexe moléculaire non-covalent, appelé IgG/ZZ-Tat_{22-57C22-37)S} et un complexe IgG/ZZ.

Des CMSP humaines préparées comme décrit dans l'exemple 7 ont ensuite été incubées en absence ou en présence des composés suivants : anti-DEC205/ZZ-Tat_{22-57C22-37)S}, IgG/ZZ-Tat_{22-57C22-37)S}, anti-DEC205/ZZ, IgG/ZZ ZZ-DTRBD_{HEK}, et de l'Ac anti-DEC205 et l'IgG libres (concentration finale de 0,1µM pour chaque composé). Dans ces expériences des CMSP ont aussi été incubées avec les protéines ZZ-Tat_{22-57C22-37)S} et ZZ libres à une concentration de 1µM. Après 24h, les cellules ont été récoltées, marquées à l'aide d'Ac fluorescents permettant d'identifier les monocytes (CD14, anti-CD14-BV605, Biolegend), les lymphocytes T-CD4+ (CD4, anti-CD4-PerCP-Cy5.5, Biolegend) et la molécule CD69 (anti-CD69-BV785, Biolegend). Après 30 minutes d'incubation, les CMSP ont été fixées à l'aide d'une solution contenant 4% de paraformaldéhyde puis analysées par cytométrie de flux.

### Résultats

Les exemples précédents montrent que des complexes moléculaires ciblant les Ac peuvent induire efficacement certains mécanismes de réponse immunitaire et ralentir efficacement la croissance tumorale. Les Ac localisées à la surface des CPAg sont les molécules ciblées par ces complexes. Les CPAg expriment cependant un grand nombre d'autres molécules pouvant aussi représenter des cibles pour des complexes moléculaires selon l'invention. Les inventeurs se sont donc demandés si des complexes moléculaires ciblant les HS et d'autres récepteurs que les immunoglobulines localisées à la surface CPAg peuvent aussi activer ces cellules. Pour évaluer cet aspect, ils ont choisi d'évaluer deux types de récepteurs. Le premier est la protéine DEC205, qui est une lectine sélectivement exprimée chez l'homme par les monocytes et certaines populations de cellules dendritiques (Kato M. et al. 2006, Int. Immunol., 18 :857-869). Le second est le récepteur FC_{gamma} dont la plupart des formes sont exprimés par les monocytes et certaines populations de cellules dendritiques.

Les inventeurs ont ensuite préparé deux complexes moléculaires pouvant respectivement cibler ces récepteurs. Pour cibler la protéine DEC205 ils ont utilisé un Ac monoclonal spécifique de ce récepteur qu'ils ont complexé à la protéine de fusion ZZ-Tat_{22-57C22-37)S}. Ils ont appelé ce complexe moléculaire anti-DEC205/ZZ-Tat_{22-57C22-37)S}. Pour cibler les récepteurs Fc ils ont utilisé un Ac IgG polyclonal humain non-spécifique qui peut interagir avec ces récepteurs via son domaine Fc. Ils ont formé un complexe moléculaire entre cette IgG et ZZ-Tat_{22-57C22-37)S}, appelé IgG/ZZ-Tat_{22-57C22-37)S}. Ces deux complexes moléculaires précités possèdent ainsi la capacité à lier des récepteurs de CPAg et les héparanes sulfates via le domaine Tat_{22-57C22-37)S} de ZZ-Tat_{22-57C22-37)S}. Dans ces complexes moléculaires, les deux protéines sont associées de façon non-covalente par l'intermédiaire de leurs domaines Fc et ZZ respectifs. ZZ ne peut donc plus cibler les immunoglobulines localisées à la surface des CPAg car il est déjà engagé dans l'interaction avec les Ac. Les inventeurs ont aussi préparé deux complexes, respectivement appelés anti-DEC205/ZZ et IgG/ZZ, dépourvus de la région Tat_{22-57C22-37)S} de liaison aux héparanes sulfates afin de les utiliser à titre de contrôle dans les expériencces d'activation ultérieures.

Comme la molécule DEC205 et les récepteurs Fc sont exprimés par les monocytes, les inventeurs se sont ensuite demandés si les complexes moléculaires peuvent permettre l'activation de cette sous-population de CPAg. Pour cela, ils ont incubé des CMSP en absence ou en présence d'une concentration fixe (0,1 µM) d'anti-DEC205/ZZ-Tat_{22-57C22-37)S}, IgG/ZZ-Tat_{22-57C22-37)S}, anti-DEC205/ZZ, IgG/ZZ, anti-DEC205, IgG, respectivement. Ils ont aussi incubé les protéines ZZ-Tat_{22-57C22-37)S} et ZZ libres à une concentration de 1µM pour évaluer l'effet de ZZ-Tat_{22-57C22-37)S} à une concentration identique à celle qui s'avère activatrice dans les exemples 3, 4 et 5. Après 24h, ils ont évalué la proportion de monocytes et de lymphocytes T-CD4+ activés. Comme on peut le voir sur la **Fig. 12A****,** ZZ ne modifie pas la proportion de monocytes activés dans les CMSP alors qu'elle est significativement accrue par ZZ-Tat_{22-57C22-37)S} ce qui indique que ce complexe moléculaire permet d'activer des CPAg humaines. La proportion de cellules activées est pour sa part diminuée avec l'Ac anti-DEC205 libre ou inclus dans le complexe anti-DEC205/ZZ. En revanche, la proportion de monocytes activés est accrue en présence du complexe anti-DEC205/ZZ-Tat_{22-57C22-37)S}. Un comportement similaire est observé lorsque les lymphocytes T-CD4+ sont considérés **(****Fig. 12B****).** Ces données indiquent donc qu'un Ac ciblant des CPAg et dépourvu de la capacité à les activer peut devenir capable de les induire quand il est inclus dans un complexe moléculaire permettant de cibler aussi les HS.

L'analyse de l'état d'activation des monocytes après incubation avec l'IgG humaine libre montre que cet Ac permet d'augmenter la proportion de monocytes activés. Le complexe IgG/ZZ ne permet pas un accroissement significatif de la proportion de cellules activées. En revanche, le nombre de monocytes activés est plus de deux fois supérieur lorsque les CMSP sont incubées avec le complexe moléculaire IgG/ZZ-Tat_{22-57C22-37)S}. Un comportement similaire est observé lorsque les lymphocytes T-CD4+ sont considérés (Fig. 12B). Ces données indiquent donc qu'un Ac ciblant des récepteurs Fc_{gamma} à la surfaces des CPAg s'avère capable de les activer mais que l'effet d'activation est accru quand l'Ac est inclus dans un complexe moléculaire permettant de cibler aussi les HS.

De façon intéressante, ces résultats montrant une augmentation conjointe de la proportion de monocytes et de lymphocytes T activés indiquent que les complexes moléculaires induisent plusieurs acteurs cellulaires qui jouent un rôle central dans les mécanismes de défense immunitaire.

### Exemple 10 : Des complexes moléculaires ciblant les cellules dendritiques induisent in vitro la sécrétion d'IL-6 par des cellules du système immunitaire.

### Matériels et Méthodes

Pour préparer des complexes moléculaires ciblant les DCs, trois anticorps spécifiques de molécules exprimées par les DCs ont été utilisés. Le premier Ac (BD référence 555538), appelé Ac anti-CD74, cible la molécule CD74. Le second Ac (BD référence 551186), appelé Ac anti-CD209, cible la molécule CD209. Le troisième Ac (BD référence 552501), appelé Ac anti-CD275, cible la molécule CD275.

Chacun de ces trois Ac (30nM pour chaque molécule) a été incubé en absence ou en présence de ZZ-Tat_{22-57C22-37)S} ou ZZ (5nM pour chaque molécule), pendant 24 heures à 4°C en milieu RPMI, 5% Sérum humain AB. La capacité de la région ZZ à lier la région Fc de ces Ac a permis de former différents complexes moléculaires non-covalent appelé anti-CD74/ZZ-Tat_{22-57C22-37)S}, anti-CD209/ZZ-Tat_{22-57C22-37)S}, anti-CD275/ZZ-Tat_{22-57C22-37)S}, anti-CD74/ZZ, anti-CD209/ZZ, et anti-CD275/ZZ, respectivement.

Des CMSPs humaines sont resuspendues à raison de 5.10⁶ cellules/mL en milieu RPMI 5% Sérum humain AB. 100 µL de la suspension cellulaire a été réparti en plaque 96 puits en présence ou en absence des Acs, ZZ ou ZZ-Tat_{22-57C(22-37)S} libre ainsi que des complexes moléculaires. Après 24h d'incubation, les surnageants sont récoltés pour un dosage de la cytokine IL-6 par ELISA réalisé selon les instructions du fabricant (R&D #DY406-05).

### Résultats

Les inventeurs se sont demandés si des complexes moléculaires ciblant trois molécules exprimées par des DCs peuvent induire l'activation de cellules du système immunitaire. Comme l'activation cellulaire peut conduire à la sécrétion de cytokines, les inventeurs ont décidé d'évaluer *in vitro* la présence d'IL-6. Ils ont trouvé cette cytokine dans les surnageants issus de l'incubation avec ZZ-Tat_{22-57C22-37)S} mais pas dans ceux issus de l'incubation avec ZZ. Ils n'en ont pas trouvé dans les surnageants issus de l'incubation des CMSPS avec l'Ac anti-CD74 libre **(****Fig. 13A****)** ce qui indique que cet Ac ne peut induire l'activation lorsqu'il est sous forme libre. Les inventeurs ont observé que la sécrétion d'IL-6 est accrue quand les CMSPs sont incubées avec le complexe anti-CD74/ZZ ce qui indique que le double domaine ZZ permet l'activation médiée par cet Ac (Fig. **13A**). Cependant, la sécrétion de cette cytokine est encore plus forte avec anti-CD74/ZZ-Tat_{22-57C22-37)S}, ce qui indique que l'ajout de la région de Tat permettant de lier les HSPGs permet d'accroître l'activation cellulaire médiée par ce complexe.

Les inventeurs se sont aperçus que les Ac anti-CD209 et anti-CD275 libres sont pour leur part capables d'induire la sécrétion d'IL-6 quand ils sont incubés avec des CMSPs (Fig. **13B,** et **13C****).** Quand ces deux Ac sont respectivement complexés à ZZ (anti-CD209/ZZ et anti-CD275/ZZ) la sécrétion de cette cytokine est aussi observée. Cette sécrétion est cependant accrue quand ces deux Ac sont complexés à ZZ-Tat_{22-57C22-37)S}, ce qui indique que le pouvoir activateur peut être augmenté quand les complexes moléculaires de ciblage sont capables de lier les HSPGs.

### Exemple 11 : Un complexe moléculaire dirigé contre le récepteur CD335 exprimé par les cellules NK et NKT induit in vitro une augmentation de la proportion de cellules NK et NKT activées.

### Matériels et Méthodes

Pour préparer des complexes moléculaires ciblant la molécule CD335 à la surface des cellules NK et NKT un Ac (Origene AM31284AF-N), appelé Ac anti-CD335 a été utilisé. Cet Ac a été incubé à 30nM en absence ou en présence de ZZ-Tat_{22-57C22-37)S} ou ZZ (5nM pour chaque molécule), pendant 5 heures à 37°C en milieu RPMI, 5% Sérum humain AB. La capacité de la région ZZ à lier la région Fc de ces Ac a permis de former deux complexes moléculaires appelés anti-CD335/ZZ-Tat_{22-57C22-37)S}, et anti-CD335/ZZ, respectivement.

Des CMSPs humaines ont été resuspendues à raison de 5.10⁶ cellules/ml en milieu RPMI 5% Sérum humain AB. 100 µL de la suspension cellulaire a été réparti en plaque 96 puits en présence ou en absence de l'Ac anti-CD335, ZZ ou ZZ-Tat_{22-57C(22-37)S} libres ainsi que des complexes moléculaires. Après 18h, les cellules ont été récoltées, marquées à l'aide d'Ac fluorescents permettant d'identifier les cellules NK (CD56+, biolegend référence 362510 dilution au 1/100), NKT (CD56+ biolegend référence 362510 ; CD3+ ; Miltenyi réf 130-113-136 dilution au 1/200), et la molécule CD69 (biolegend référence 310932, dilution au 1/100). Après 30 minutes d'incubation, les CMSP ont été fixées à l'aide d'une solution contenant 4% de paraformaldéhyde puis analysées par cytométrie de flux

### Résultats

Les inventeurs se sont demandés si des complexes moléculaires ciblant la molécule CD335 exprimée à la surface des cellules NK et NKT peuvent induire l'activation de ces deux types cellulaires. Pour évaluer cela ils ont utilisé l'Ac anti-CD335 sous forme isolée ou incluse dans un complexe moléculaire. Ils ont incubé des CMSP en absence ou en présence des différents mélanges. Comme les cellules NK et NKT jouent un rôle crucial dans les mécanismes de défense immunitaire, après 18h d'incubation, ils ont évalué la proportion de cellules NK et NKT activés en suivant l'expression de la molécule de co-stimulation CD69.

Comme on peut le voir sur la figure **14****,** ZZ, l'Ac anti-CD335 isolé et le complexe anti-CD335/ZZ n'augmentent pas la proportion de cellules NK **(A)** ou NKT **(B)** exprimant CD69 dans les CMSP. Un comportement différent est observé avec ZZ-Tat_{22-57C22-37)S} libre. En effet, ce complexe moléculaire n'augmente pas la proportion de cellules NKT exprimant CD69 mais accroît en revanche le pourcentage de cellules NK exprimant ce marqueur ce qui indique que l'association de ZZ et du ligand des HS dans le complexe ZZ-Tat_{22-57C(22-37)S} permet d'accroître l'activation des cellules NK. Le complexe moléculaire anti-CD335/ZZ-Tat_{22-57C(22-37)S}, à pour sa part un impact sur l'activation des deux sous-populations cellulaires. En effet, il augmente la proportion de cellules NK et NKT exprimant CD69. L'ensemble de ces données indique donc que l'Ac anti-CD335 est dépourvu de la capacité à activer les deux types cellulaires mais peut devenir capable de les induire quand il est inclus dans un complexe moléculaire permettant de cibler aussi les HS.

### Exemple 12 : Des complexes moléculaires respectivement dirigés contre les molécules CD56 (A) et CD336 (B) exprimés par les cellules NK et NKT induisent in vitro une augmentation de la proportion de cellules NKT activées.

### Matériels et Méthodes

Pour préparer des complexes moléculaires ciblant les molécules CD56 et CD336 à la surface des cellules NK et NKT l'Ac anti-CD56 (Biolegend référence 304622) qui cible la molécule CD56 et l'Ac anti-CD336 (Origene AM50346PU-N) qui cible la molécule CD336 ont été utilisés.

Ces Ac ont été incubés à 30nM en absence ou en présence de ZZ-Tat_{22-57C22-37)S} ou ZZ (5nM pour chaque molécule), pendant 5 heures à 37°C en milieu RPMI, 5% Sérum humain AB. La capacité de la région ZZ à lier la région Fc de ces Ac a permis de former quatre complexes moléculaires appelés respectivement anti-CD56/ZZ-Tat_{22-57C22-37)S}, anti-CD56/ZZ, anti-CD336/ZZ-Tat_{22-57C22-37)S}, et anti-CD336/ZZ.

Des CMSPs humaines ont été resuspendues à raison de 5.10⁶ cellules/ml en milieu RPMI 5% Sérum humain AB. 100 µL de la suspension cellulaire a été réparti en plaque 96 puits en présence ou en absence des formes isolées de l'Ac anti-CD56, Ac anti-CD336, ZZ ou ZZ-Tat_{22-57C(22-37)S} ainsi que des complexes moléculaires non-covalents. Après 18h, les cellules ont été récoltées, marquées à l'aide d'Ac fluorescents décrits dans l'exemple 11 qui permettent d'identifier les cellules NKT (CD56+CD3+), et la molécule CD69. Après 30 minutes d'incubation, les CMSP ont été fixées à l'aide d'une solution contenant 4% de paraformaldéhyde puis analysées par cytométrie de flux.

### Résultats

Les inventeurs se sont demandés si des complexes moléculaires ciblant les molécule CD56 et CD336 exprimées peuvent induire l'activation des cellules NKT. Pour évaluer cela ils ont utilisé les Ac anti-CD56 et anti-CD336 sous forme isolée ou incluse dans un complexe moléculaire. Ils ont incubé des CMSP en absence ou en présence des différents mélanges. Après 18h d'incubation, ils ont évalué la proportion de cellules NKT activés en suivant l'expression de la molécule de co-stimulation CD69.

Comme on peut le voir sur la figure **15****,** ZZ, ZZ-Tat_{22-57C22-37)S} , l'Ac anti-CD56, l'Ac anti-CD336 isolé et le complexe anti-CD336/ZZ n'augmentent pas la proportion de cellules NKT exprimant CD69 dans les CMSP. Un comportement différent est observé avec les complexes moléculaires anti-CD56/ZZ-Tat_{22-57C22-37)S} (fig. **15A**), anti-CD336/ZZ-Tat_{22-57C22-37)S}. (fig. **15B**). En effet, une augmentation de la proportion de cellules NKT exprimant CD69 est trouvée lorsque ces deux complexes moléculaires sont incubés avec des CMSP. L'ensemble de ces données indique donc que l'Ac anti-CD56, et Ac anti-CD336 isolés sont dépourvus de la capacité à activer les cellules NKT mais peuvent devenir capable de les induire quand ils sont inclus dans un complexe moléculaire ciblant aussi les HS.

### Exemple 13 : Des complexes moléculaires ciblant les cellules NK et NKT induisent in vitro la sécrétion d'IL-6 par des cellules du système immunitaire.

### Matériels et Méthodes

Pour préparer des complexes moléculaires ciblant les cellules NK et NKT les trois anticorps décrits dans les exemples 10 et 11 ont été utilisés. Chacun de ces trois Ac (30nM pour chaque molécule) a été incubé en absence ou en présence de ZZ-Tat_{22-57C22-37)S} ou ZZ (5nM pour chaque molécule), pendant 24 heures en milieu RPMI, 5% Sérum humain AB. La capacité de la région ZZ à lier la région Fc de ces Ac a permis de former différents complexes moléculaires non-covalent appelés anti-CD56/ZZ-Tat_{22-57C22-37)S}, anti-CD335/ZZ-Tat_{22-57C22-37)S}, anti-CD336/ZZ-Tat_{22-57C22-37)S}. anti-CD56/ZZ, anti-CD335/ZZ, et anti-CD336/ZZ, respectivement.

Des CMSPs humaines sont resuspendues à raison de 5.10⁶ cellules/mL en milieu RPMI 5% Sérum humain AB. 100 µL de la suspension cellulaire sont répartis en plaque 96 puits en présence ou en absence des Acs, ZZ ou ZZ-Tat_{22-57C(22-37)S} libre ainsi que des complexes moléculaires. Après 24h d'incubation, les surnageants sont récoltés pour un dosage de la cytokine IL-6 par ELISA réalisé selon les instructions du fabricant (R&D #DY406-05).

### Résultats

Les inventeurs se sont demandés si des complexes moléculaires ciblant les cellules NK et NKT peuvent induire l'activation de cellules du système immunitaire. Comme l'activation cellulaire peut conduire à la sécrétion de cytokines, les inventeurs ont décidé d'évaluer *in vitro* la présence d'IL-6. Ils ont trouvé cette cytokine dans les surnageants issus de l'incubation avec ZZ-Tat_{22-57C22-37)S} mais pas dans ceux issus de l'incubation avec ZZ. Ils n'ont pas trouvé d'IL-6 dans les surnageants issus de l'incubation des CMSPS avec les Ac anti-CD56 et anti-CD335 libres **(****Fig. 16A et 16B****),** ce qui indique que ces deux protéines ne peuvent induire l'activation lorsqu'ils sont sous forme libre. Ils ont en revanche détecté la cytokine dans les surnageants issus de l'incubation avec l'anticorps anti-CD336 **(****Fig. 16C****)** ce qui indique que, sous sa forme libre, cet Ac est doté d'une activité stimulante. Les inventeurs ont observé, pour les trois Ac, une sécrétion d'IL-6 accrue quand les CMSPs sont respectivement incubées avec les complexes anti-CD56/ZZ-Tat_{22-57C22-37)S}, **(****Fig. 16A****),** anti-CD335/ZZ-Tat_{22-57C22-37)S}, **(****Fig. 16B****)** et anti-CD336/ZZ-Tat_{22-57C22-37)S} **(****Fig. 16C****).** Ces données indiquent donc que des complexes moléculaires ciblant les cellules NK et les HSPGs peuvent induire une activation efficace de cellules immunitaires.

### Exemple 14 : Des complexes moléculaires ciblant des points de contrôle de la réponse immunitaire (ICP) induisent in vitro la sécrétion d'IL-6 par des cellules du système immunitaire.

### Matériels et Méthodes

Pour préparer des complexes moléculaires ciblant des ICPs trois anticorps spécifiques de molécules considérées comme des ICPs ont été utilisés. Le premier Ac (BioXCell référence BE0190), appelé Ac anti-CTLA-4, cible la molécule CTLA-4. Le second Ac (BioXCell référence BE0285), appelé Ac anti-PD-L1, cible la molécule PD-L1. Le troisième Ac (R&D référence MAB10542), appelé Ac anti-OX40, cible la molécule OX40.

Chacun de ces trois Ac (30nM pour chaque molécule) a été incubé en absence ou en présence de ZZ-Tat_{22-57C22-37)S} ou ZZ (5nM pour chaque molécule), pendant 24 heures à 4°C en milieu RPMI, 5% Sérum humain AB. La capacité de la région ZZ à lier la région Fc de ces Ac a permis de former différents complexes moléculaires non-covalent, appelé anti-CTLA-4/ZZ-Tat_{22-57C22-37)S}, anti-PD-L1/ZZ-Tat_{22-57C22-37)S}, anti-OX40/ZZ-Tat_{22-57C22-37)S}, anti-CTLA-4/ZZ, anti-PD-L1/ZZ, et anti-OX40/ZZ, respectivement.

Des CMSPs humaines ont été resuspendues à raison de 5.10⁶ cellules/mL en milieu RPMI 5% Sérum humain AB. 100 µL de la suspension cellulaire a été répartie en plaque 96 puits en présence ou en absence des Ac, ZZ ou ZZ-Tat_{22-57C(22-37)S} libres ainsi que des complexes moléculaires. Après 24h d'incubation, les surnageants ont été récoltés pour un dosage de la cytokine IL-6 par ELISA réalisé selon les instructions du fabricant (R&D #DY406-05).

### Résultats

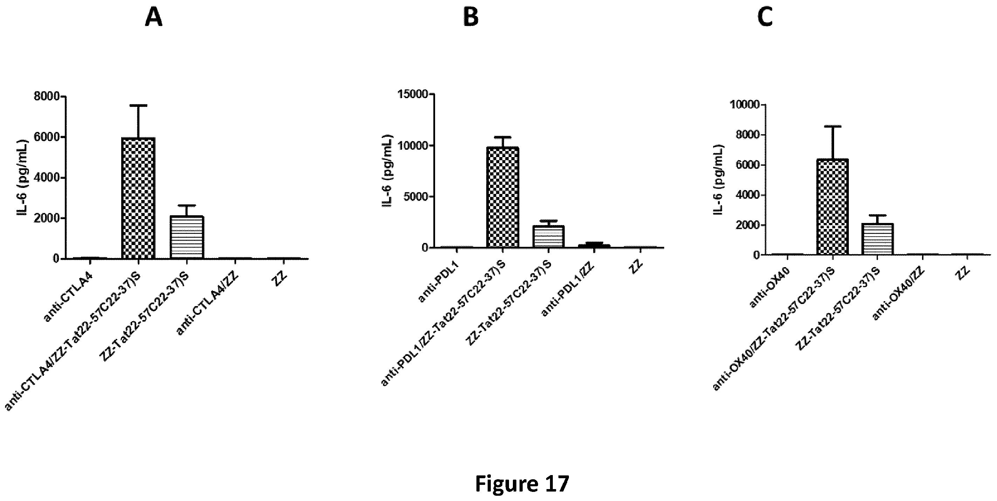

Les inventeurs se sont demandés si des complexes moléculaires ciblant des ICPs peuvent induire l'activation de cellules du système immunitaire. Comme l'activation cellulaire peut conduire à la sécrétion de cytokines, les inventeurs ont décidé d'évaluer *in vitro* la présence d'IL-6. Ils n'en ont pas trouvé dans les surnageants issus de l'incubation des CMSPS avec les trois différents Ac libres ou avec ZZ libre ce qui indique que ces composés ne peuvent induire l'activation lorsqu'ils sont sous forme libre **(****Fig. 17A, 17B, et 17C****).** En revanche, ils ont trouvé cette cytokine dans les surnageants issus de l'incubation avec ZZ-Tat_{22-57C22-37)S} ce qui démontre que l'association du double domaine de liaison aux Ig et du domaine Tat_{22-57C22-37)S} de liaison aux HS permet l'activation de cellules du système. Les inventeurs ont cependant observé que la sécrétion d'IL-6 est encore accrue quand les CMSPs sont respectivement incubées avec les complexes anti-CTLA-4/ZZ-Tat_{22-57C22-37)S}, **(****Fig. 17A****),** anti-PD-L1/ZZ-Tat_{22-57C22-37)S}, **(****Fig. 17B****),** anti-OX40/ZZ-Tat_{22-57C22-37)S} **(****Fig. 17C****).** En revanche, l'IL-6 est absente des surnageants issus de l'incubation avec anti-CTLA-4/ZZ, anti-PD-L1/ZZ, ou anti-OX40/ZZ ce qui indique que le double domaine ZZ ne contribue pas à l'activation médiée par ces Ac. L'ensemble de ces données indique donc que des complexes moléculaires ciblant des ICPs et les HSPGs peuvent induire une activation de cellules immunitaires.

## Revendications

1. Complexe moléculaire pour utilisation en tant que médicament immunostimulant en immunothérapie du cancer ou des maladies infectieuses, ledit complexe consistant en au moins un ligand d'un sucre sulfaté de la famille des glycosaminoglycanes exprimé à la surface de cellules présentatrices d'antigène ou de cellules NK ou NKT (premier ligand) et au moins un ligand d'une molécule de surface de cellules présentatrices d'antigène ou de cellules NK ou NKT autre qu'un sucre sulfaté de la famille des glycosaminoglycanes (deuxième ligand) liés entre eux, et ledit complexe étant dépourvu d'un antigène spécifique de la maladie à traiter.

2. Complexe moléculaire pour utilisation selon le revendication 1, dans lequel le premier ligand est un peptide de liaison des héparanes sulfates sélectionné dans le groupe constitué par : un peptide issu de la protéine Tat du VIH comprenant au moins la région basique Tat49-57 (SEQ ID NO : 3) tel que les peptides Tat49-57 (SEQ ID NO: 3), Tat37-57 (SEQ ID NO : 8) et Tat22-57C_{(22-37)S} (SEQ ID NO :9) ; un peptide polyarginine R7 à R11 ; et un peptide comprenant le domaine R de la toxine diphtérique (SEQ ID NO : 5) ou au moins le fragment DT453-467 (SEQ ID NO 7) dudit domaine comprenant la région de liaison des héparanes sulfates.

3. Complexe moléculaire pour utilisation selon l'une quelconque des revendications précédentes, dans lequel le deuxième ligand cible une molécule de surface de cellules présentatrices d'antigène sélectionnée dans le groupe constitué par : les récepteurs lectines de type C, les immunoglobulines membranaires, les récepteurs pour la région constante des immunoglobulines, et les molécules de points de contrôle immunitaire et leurs ligands.

4. Complexe moléculaire pour utilisation selon l'une quelconque des revendications précédentes, dans lequel le deuxième ligand est sélectionné dans le groupe constitué par : (i) les anticorps dirigés contre lesdites molécules de surface des cellules présentatrices d'antigène ou des cellules NK ou NKT et leurs fragments contenant au moins le paratope ; (ii) les immunoglobulines de préférence les IgG, et leurs fragments comprenant au moins la région Fc ; et (iii) les protéines et les fragments de protéines qui lient la région Fc et/ou Fab des anticorps, notamment la protéine A de *S*. *aureus,* son fragment BB (SEQ ID NO : 1) et son dérivé ZZ (SEQ ID NO : 2).

5. Complexe moléculaire pour utilisation selon l'une quelconque des revendications précédentes, sous forme d'oligomères ou d'un mélange de monomères et d'oligomères.

6. Complexe moléculaire pour utilisation selon l'une quelconque des revendications précédentes, consistant en une protéine de fusion entre le premier et le deuxième ligand.

7. Complexe moléculaire pour utilisation selon la revendication 6, consistant en une protéine de fusion comprenant un premier ligand choisi parmi : Tat49-57 (SEQ ID NO : 3), Tat37-57 (SEQ ID NO : 8), Tat22-57C(22-37)S (SEQ ID NO 9), le domaine R de la toxine diphtérique (SEQ ID NO : 5) ou le fragment DT453-467 (SEQ ID NO 7) et un deuxième ligand choisi parmi : le fragment BB de la protéine A (SEQ ID NO : 1) ou son dérivé ZZ (SEQ ID NO : 2).

8. Complexe moléculaire pour utilisation selon l'une quelconque des revendications 1 à 5, dans lequel le deuxième ligand est un anticorps ou un fragment d'anticorps et le premier ligand forme une protéine de fusion avec un élément de liaison aux immunoglobulines, de préférence la protéine A de *S*. *aureus,* son fragment BB (SEQ ID NO : 1) ou son dérivé ZZ (SEQ ID NO : 2).

9. Complexe moléculaire pour utilisation selon la revendication 8, dans lequel ladite protéine de fusion comprend un premier ligand choisi parmi Tat49-57 (SEQ ID NO : 3), Tat37-57 (SEQ ID NO : 8), Tat22-57C(22-37)S (SEQ ID NO 9), le domaine R de la toxine diphtérique (SEQ ID NO : 5) ou le fragment DT453-467 (SEQ ID NO 7) et un élément de liaison aux immunoglobulines choisi parmi le fragment BB de la protéine A (SEQ ID NO : 1) ou son dérivé ZZ (SEQ ID NO : 2).

10. Complexe moléculaire pour utilisation selon la revendication 9, dans lequel l'élément de liaison aux immunoglobulines est le fragment BB de la protéine A (SEQ ID NO : 1), et ladite protéine de fusion étant complexée au deuxième ligand qui consiste en une immunoglobuline entière.

11. Complexe moléculaire pour utilisation selon la revendication 8, dans lequel ladite protéine de fusion est complexée au deuxième ligand qui est choisi parmi un anticorps anti-RFcgamma I, Il et/ou III, anti-DEC-205, anti-DC-SIGN, anti-CD74, anti-CD275, anti-CD56, anti-CD335, anti-CD336, anti-CTLA-4, anti-PD-L1, anti-OX40, ou un fragment des anticorps précédents comprenant au moins le paratope.

12. Complexe moléculaire pour utilisation selon l'une quelconque des revendications précédentes en tant que médicament immunostimulant, de préférence pour activer des cellules présentatrices de l'antigène, notamment des cellules dendritiques ou des monocytes, pour activer des cellules NK ou NKT, et/ou pour activer la sécrétion des cytokines IL-6 et/ou IL-12.

13. Composition pour utilisation en tant que médicament immunostimulant en immunothérapie du cancer ou des maladies infectieuses, comprenant au moins un complexe moléculaire tel que défini à l'une quelconque des revendications précédentes, et au moins un véhicule pharmaceutiquement acceptable, une substance porteuse et/ou un adjuvant.

14. Composition pour utilisation selon la revendication 13, dans laquelle l'adjuvant est un oligodéoxynucléotide CpG, de l'acide polyinosinique-polycytidylique ou un mélange d'oligodéoxynucléotide(s) CpG, et d'acide polyinosinique-polycytidylique, et/ou la substance porteuse est une nanoparticule.

15. Composition pour utilisation selon la revendication 13 ou 14, comprenant au moins un autre agent thérapeutique, de préférence au moins un inhibiteur de point de contrôle immunitaire, de manière préférée un anti-PD-1, un anti-PDL-1 ou un anti-CTLA4.

## Patentansprüche

1. Molekularkomplex zur Verwendung als immunstimulierendes Arzneimittel zur Immuntherapie von Krebs oder Infektionskrankheiten, wobei der Komplex aus mindestens einem Liganden eines sulfatierten Zuckers der Familie der Glykosaminoglykane besteht, der an der Oberfläche von Antigen-präsentierenden Zellen oder NK- oder NKT-Zellen exprimiert wird (erster Ligand) und mindestens einen Liganden eines Oberflächenmoleküls von Antigen-präsentierenden Zellen oder von NK- oder NKT-Zellen, der kein sulfatierter Zucker der Familie der miteinander verbundenen Glykosaminoglykane (zweiter Ligand) ist, und wobei der Komplex frei von einem Antigen ist, das für die zu behandelnde Krankheit spezifisch ist.

2. Molekularkomplex zur Verwendung nach Anspruch 1, wobei der erste Ligand ein Heparansulfat-Bindungspeptid ist, ausgewählt aus der Gruppe, bestehend aus: einem Peptid aus dem HIV-Protein Tat, das mindestens den Basisbereich Tat49-57 (SEQ ID-NR: 3) wie die Peptide Tat49-57 (SEQ ID-NR: 3), Tat37-57 (SEQ ID-NR: 8) und Tat22-57C_{(22-37)S} (SEQ ID-NR: 9); ein Polyarginin-Peptid R7 bis R11; und ein Peptid umfasst, umfassend die R-Domäne des Diphtherietoxins (SEQ ID-NR: 5) oder mindestens das Fragment DT453-467 (SEQ ID-NR 7) der Domäne, der den Heparansulfat-Bindungsbereich umfasst.

3. Molekularkomplex zur Verwendung nach einem der vorhergehenden Ansprüche, wobei der zweite Ligand auf ein Oberflächenmolekül von Antigen-präsentierenden Zellen abzielt, das aus der Gruppe ausgewählt ist, bestehend aus: Lektin-Typ-C-Rezeptoren, membranförmigen Immunglobulinen, Rezeptoren für den Immunglobulin-Konstantbereich und Immun-Checkpoint-Molekülen und deren Liganden.

4. Molekularkomplex zur Verwendung nach einem der vorhergehenden Ansprüche, wobei der zweite Ligand aus der Gruppe ausgewählt ist, bestehend aus: (i) Antikörper gegen die Oberflächenmoleküle von Antigen-präsentierenden Zellen oder NK- oder NKT-Zellen und deren Fragmente, die mindestens den Paratop enthalten; (ii) Immunglobuline, vorzugsweise IgG, und Fragmente davon, die mindestens den Fc-Bereich umfassen; und (iii) Proteine und Proteinfragmente, die den Fc- und/oder Fab-Bereich der Antikörper binden, insbesondere das Protein A von ***S.** aureus,* sein BB-Fragment (SEQ ID-NR: 1) und sein Derivat ZZ (SEQ ID-NR: 2).

5. Molekularkomplex zur Verwendung nach einem der vorhergehenden Ansprüche, in Form von Oligomeren oder einer Mischung aus Monomeren und Oligomeren.

6. Molekularkomplex zur Verwendung nach einem der vorhergehenden Ansprüche, bestehend aus einem Fusionsprotein zwischen dem ersten und dem zweiten Liganden.

7. Molekularkomplex zur Verwendung nach Anspruch 6, bestehend aus einem Fusionsprotein, das einen ersten Liganden umfasst, der aus Folgendem ausgewählt ist: Tat49-57 (SEQ ID-NR: 3), Tat37-57 (SEQ ID-NR: 8), Tat22-57C(22-37)S (SEQ ID-NR 9), der R-Domäne des Diphtherietoxins (SEQ ID-NR: 5) oder dem Fragment DT453-467 (SEQ ID-NR 7), und ein zweiter Ligand, der aus Folgendem ausgewählt ist: dem BB-Fragment des Proteins A (SEQ ID-NR: 1) oder seinem Derivat ZZ (SEQ ID-NR: 2).

8. Molekularkomplex zur Verwendung nach einem der Ansprüche 1 bis 5, wobei der zweite Ligand ein Antikörper oder ein Antikörperfragment ist und der erste Ligand ein Fusionsprotein mit einem Immunglobulin-Bindungselement bildet, vorzugsweise dem Protein A von *S. aureus,* dessen BB-Fragment (SEQ ID-NR: 1) oder seinem Derivat ZZ (SEQ ID-NR: 2).

9. Molekularkomplex zur Verwendung nach Anspruch 8, wobei das Fusionsprotein einen ersten Liganden umfasst, der aus Tat49-57 (SEQ ID-NR: 3), Tat37-57 (SEQ ID-NR: 8), Tat22-57C(22-37)S (SEQ ID-NR 9), R-Domäne des Diphtherietoxins (SEQ ID-NR: 5) oder dem Fragment DT453-467 (SEQ ID-NR 7) ausgewählt ist und einem Immunglobulin-Bindungselement, ausgewählt aus dem BB-Fragment des Proteins A (SEQ ID-NR: 1) oder seinem Derivat ZZ (SEQ ID-NR: 2).

10. Molekularkomplex zur Verwendung nach Anspruch 9, wobei das Immunglobulin-Bindungselement das BB-Fragment des Proteins A ist (SEQ ID-NR: 1), wobei das Fusionsprotein mit dem zweiten Liganden komplexiert ist, der aus einem ganzen Immunglobulin besteht.

11. Molekularkomplex zur Verwendung nach Anspruch 8, wobei das Fusionsprotein mit dem zweiten Liganden komplexiert ist, der aus einem Anti-RFcgamma I, II und/oder III, Anti-DEC-205-Antikörper, Anti-DC-SIGN, Anti-CD74, Anti-CD275, Anti-CD56, Anti-CD335, Anti-CD336, Anti-CTLA-4, Anti-PD-L1, Anti-OX40 oder einem Fragment der vorhergehenden Antikörper ausgewählt ist, das mindestens den Paratop umfasst.

12. Molekularkomplex zur Verwendung nach einem der vorhergehenden Ansprüche als immunstimulierendes Arzneimittel, vorzugsweise zur Aktivierung von Antigen-präsentierenden Zellen, insbesondere dendritischen Zellen oder Monozyten, zur Aktivierung von NK- oder NKT-Zellen und/oder zur Aktivierung der Sekretion der Zytokine IL-6 und/oder IL-12.

13. Zusammensetzung zur Verwendung als immunstimulierendes Arzneimittel zur Immuntherapie von Krebs oder Infektionskrankheiten, umfassend mindestens einen Molekularkomplex nach einem der vorhergehenden Ansprüche und mindestens ein pharmazeutisch akzeptables Trägermedium, eine Trägersubstanz und/oder ein Adjuvant.

14. Zusammensetzung zur Verwendung nach Anspruch 13, wobei der Adjuvant ein Oligodeoxynukleotid CpG, Polyinosin-Polycytidylsäure oder eine Mischung aus Oligodeoxynukleotid(en) CpG und Polyinosin-Polycytidylsäure ist, und/oder die Trägersubstanz ein Nanopartikel ist.

15. Zusammensetzung zur Verwendung nach Anspruch 13 oder 14, umfassend mindestens einen anderen therapeutischen Wirkstoff, vorzugsweise mindestens einen Immuncheckpoint-Inhibitor, vorzugsweise ein Anti-PD-1, ein Anti-PDL-1 oder ein Anti-CTLA4.

## Claims

1. Molecular complex for use as an immunostimulant medicament in the immunotherapy of cancer or infectious diseases, said complex consisting of at least one ligand of a sulphated sugar of the glycosaminoglycan family expressed on the surface of antigen-presenting cells or NK or NKT cells (first ligand) and at least one ligand of a surface molecule of antigen-presenting cells or of NK or NKT cells other than a sulphated sugar of the glycosaminoglycan family (second ligand) linked together, and said complex being devoid of an antigen specific for the disease to be treated.

2. Molecular complex for use according to claim 1, wherein the first ligand is a heparan sulphate binding peptide selected from the group consisting of: a peptide derived from the HIV Tat protein comprising at least the basic region Tat49-57 (SEQ ID NO: 3) such as peptides Tat49-57 (SEQ ID NO: 3), Tat37-57 (SEQ ID NO: 8) and Tat22-57C(22-37)S (SEQ ID NO: 9); a polyarginine peptide R7 to R11; and a peptide comprising the diphtheria toxin R domain (SEQ ID NO: 5) or at least the fragment DT453-467 (SEQ ID NO:7) of said domain comprising the heparan sulphate binding region.

3. Molecular complex for use according to any one of the preceding claims, wherein the second ligand targets an antigen-presenting cell surface molecule selected from the group consisting of: type C lectin receptors, membrane immunoglobulins, receptors for the constant region of immunoglobulins, and immune checkpoint molecules and their ligands.

4. Molecular complex for use according to any one of the preceding claims, wherein the second ligand is selected from the group consisting of: (i) antibodies directed against said surface molecules of antigen-presenting cells or NK or NKT cells and fragments thereof containing at least the paratope; (ii) immunoglobulins, preferably IgG, and fragments thereof comprising at least the Fc region; and (iii) proteins and protein fragments that bind the Fc and/or Fab region of antibodies, in particular *S. aureus* protein A, its fragment BB (SEQ ID NO: 1) and its derivative ZZ (SEQ ID NO: 2).

5. Molecular complex for use according to any one of the preceding claims, in the form of oligomers or a mixture of monomers and oligomers.

6. Molecular complex for use according to any one of the preceding claims, consisting of a fusion protein between the first and second ligand.

7. Molecular complex for use according to claim 6, consisting of a fusion protein comprising a first ligand selected from: Tat49-57 (SEQ ID NO: 3), Tat37-57 (SEQ ID NO: 8) and Tat22-57C(22-37)S (SEQ ID NO:9); the diphtheria toxin R domain (SEQ ID NO: 5) or the fragment DT453-467 (SEQ ID NO:7) and a second ligand selected from: the fragment BB of protein A (SEQ ID NO: 1) or its derivative ZZ (SEQ ID NO: 2).

8. Molecular complex for use according to any one of claims 1 to 5, wherein the second ligand is an antibody or an antibody fragment and the first ligand forms a fusion protein with an immunoglobulin binding element, preferably *S. aureus* protein A, its fragment BB (SEQ ID NO: 1) or its derivative ZZ (SEQ ID NO: 2).

9. Molecular complex for use according to claim 8, wherein said fusion protein comprises a first ligand selected from Tat49-57 (SEQ ID NO: 3), Tat37-57 (SEQ ID NO: 8) and Tat22-57C(22-37)S (SEQ ID NO: 9); the diphtheria toxin R domain (SEQ ID NO: 5) or the fragment DT453-467 (SEQ ID NO:7) and an immunoglobulin-binding element selected from the fragment BB of protein A (SEQ ID NO: 1) or its derivative ZZ (SEQ ID NO: 2).

10. Molecular complex for use according to claim 9, wherein the immunoglobulin-binding element is the fragment BB of protein A (SEQ ID NO: 1), and said fusion protein being complexed with the second ligand, which consists of a whole immunoglobulin.

11. Molecular complex for use according to claim 8, wherein said fusion protein is complexed with the second ligand, which is selected from an anti-Fc gamma RI, RII and/or RIII, anti-DEC-205 , anti-DC-SIGN, anti-CD74, anti-CD275, anti-CD56, anti-CD335, anti-CD336, anti-CTLA-4, anti-PD-L1 or anti-OX40 antibody, or a fragment of the preceding antibodies comprising at least the paratope.

12. Molecular complex for use according to any one of the preceding claims as an immunostimulant medicament, preferably to activate antigen-presenting cells, in particular dendritic cells or monocytes, to activate NK or NKT cells, and/or to activate the secretion of IL-6 and/or IL-12 cytokines.

13. Composition for use as an immunostimulant medicament in the immunotherapy of cancer or infectious diseases, comprising at least one molecular complex as defined in any one of the preceding claims, and at least one pharmaceutically acceptable vehicle, a carrier substance and/or an adjuvant.

14. Composition for use according to claim 13, wherein the adjuvant is a CpG oligodeoxynucleotide, polyinosinic-polycytidylic acid or a mixture of CpG oligodeoxynucleotide(s), and polyinosinic-polycytidylic acid, and/or the carrier substance is a nanoparticle.

15. The composition for use according to claim 13 or 14, comprising at least another therapeutic agent, preferably at least one immune checkpoint inhibitor, preferably an anti-PD-1, an anti-PDL-1 or an anti-CTLA4.
